(19) Europäisches Patentamt / European Patent Office / Office européen des brevets

(11) **EP 1 720 976 B1**

(12) # EUROPÄISCHE PATENTSCHRIFT

(45) Veröffentlichungstag und Bekanntmachung des Hinweises auf die Patenterteilung:
**08.10.2014 Patentblatt 2014/41**

(21) Anmeldenummer: **05715581.4**

(22) Anmeldetag: **26.02.2005**

(51) Int Cl.:
***C12N 1/06*** *(2006.01)*     ***C12N 15/10*** *(2006.01)*

(86) Internationale Anmeldenummer:
**PCT/EP2005/002065**

(87) Internationale Veröffentlichungsnummer:
**WO 2005/083054 (09.09.2005 Gazette 2005/36)**

(54) **VERFAHREN UND MITTEL ZUM THERMISCHEN KONDITIONIEREN EINER ZELLE**

METHOD AND DEVICE FOR THERMAL CONDITIONING OF A CELL

PROCEDES ET AGENTS DE CONDITIONNEMENT THERMIQUE D'UNE CELLULE

(84) Benannte Vertragsstaaten:
**AT BE BG CH CY CZ DE DK EE ES FI FR GB GR HU IE IS IT LI LT LU MC NL PL PT RO SE SI SK TR**

(30) Priorität: **27.02.2004 DE 102004010828**

(43) Veröffentlichungstag der Anmeldung:
**15.11.2006 Patentblatt 2006/46**

(73) Patentinhaber: **INSILICO biotechnology AG**
**70563 Stuttgart (DE)**

(72) Erfinder:
• **DAUNER, Michael**
**72147 Nehren (DE)**
• **SCHAUB, Jochen**
**70569 Stuttgart (DE)**

(74) Vertreter: **Schwahn, Hartmut et al**
**Gleiss Große Schrell und Partner mbB**
**Patentanwälte Rechtsanwälte**
**Leitzstraße 45**
**70469 Stuttgart (DE)**

(56) Entgegenhaltungen:
**US-A- 5 837 452     US-B1- 6 197 553**

**Beschreibung**

Die vorliegende Erfindung betrifft Verfahren und Mittel zum thermischen Konditionieren einer biologischen Zelle sowie Verfahren und Verwendungen dieser Mittel zur Freisetzung und Isolierung von Inhaltsstoffen aus der biologischen Zelle und zum quantitativen und qualitativen Nachweis der freigesetzten Inhaltsstoffe.

Weiter betrifft die vorliegende Erfindung ein Probennahmeverfahren, welches in einem einzigen Schritt die Überführung einer Probe biologischer Zellen aus einem Kulturmedium und die Freisetzung der Zellinhaltsstoffe bei der Überführung vorsieht, sowie Vorrichtungen zur Durchführung der Probennahme.

Vitale biologische Zellen erhalten unter physiologischen Wachstumsbedingungen ihre Zellstruktur aufrecht. Aufgrund der cytoplasmatischen Membran beziehungsweise Membranen weiterer Zellkompartimente werden aus vitalen biologischen Zellen nur Stoffinrechselendprodukte oder Botenstoffe, in der Regel nicht aber hauptanteilig die in der Zelle enthaltenden Inhaltsstoffe, insbesondere Metabolite, in die Zellumgebung abgeben. Metabolit ist jede im biologischen Stoffwechsel auftretende, im Wesentlichen niedermolekulare Substanz. Sie ist zu unterscheiden von den so genannten Biopolymeren wie Proteinen, DNA-Molekülen, mRNA-Molekülen und anderen, wobei deren Vorstufen, Abbau-, Spalt- und Bildungsprodukte jedoch wiederum den Metaboliten zuzurechnen sind.

Um Zellinhaltsstoffe und insbesondere Metabolite aus biologischen Zellen zu gewinnen, werden im Stand der Technik einige Verfahren seit langem unverändert angewandt, die jedoch gravierende Nachteile aufweisen.

Bei etablierten Verfahren wird in der Regel die zelluläre Integrität zerstört. Hierunter fallen beispielsweise die Metabolit-Freisetzung mittels Ultraschall, mechanische Zellaufschlussverfahren (z.B. Rührwerkskugelmühle) oder auch der lange bekannte Einsatz von kochendem Wasser oder von überkritischen Lösungen, zum Beispiel im Autoklaven. Biologischen Zellen setzen bekanntermaßen durch lange Erhitzung in kochendem Wasser Makromoleküle wie Plasmid-DNA in die Kulturlösung frei (Gerhardt, P., R. G. E. Murrac, W. A. Wood, and N. R. Krieg (1994) Methods for general and molecular bacteriology. American Society for Microbiology, Washington, D.C.; Sambrook, J., E. F. Fritsch, and T. Maniatis (1989) Molecular cloning: A laboratory manual, 2. ed., Cold Spring Harbor).

Andere bekannte Methoden zur Metabolit-Freisetzung, beispielsweise der Einsatz von chemischen Lösungsmitteln, Säuren, Laugen und unpolaren Lösungsmitteln, modifizieren die Zusammensetzung der Proben-Suspension derart, dass anschließend ausgewählte Detektionsmethoden, wie die Bestimmung der Metabolit-Konzentrationen durch enzymatische Assays, nicht mehr eingesetzt werden können. Oder aber werden durch die chemischen Lösungsmittel Fällungsreaktionen beziehungsweise chemische Reaktionen der Metabolite angeregt, die zu einer Verfälschung von Mess- und Analyseergebnisse führen. Wünschenswert sind daher Zellaufschlussverfahren, bei deren Anwendung die freigesetzten Inhaltsstoffe, insbesondere die Metabolite, im Wesentlichen unverändert erhalten werden können. Weitere bekannte Aufschluss- beziehungsweise Lyseverfahren sind die Einwirkung chemischer Reagenzien sowie Gefrier-Tau-Zyklen.

Bei diesen Vorgehensweisen werden die biologischen Zellen thermisch, chemisch und/oder mechanisch belastet und verlieren ihre zelluläre strukturelle Integrität beziehungsweise Zellstruktur, das heißt sie lysieren. Die Zellwand und die Cytoplasmamembran werden dabei zerstört und die Inhaltsstoffe werden freigesetzt. Dabei bilden sich auch Zelltrümmer in Form von beispielsweise Micellen, nackter DNA und Proteinkomplexen.

Nach derartigem Vorgehen liegen nach der Zelllyse nachteilhafterweise in der Zellsuspension neben den interessierenden freigesetzten Inhaltsstoffen auch Zelltrümmer, nackte DNA und Proteinkomplexe vor. Die Gegenwart dieser Komponenten ist beim Nachweis und der Analyse der Inhaltstoffe, insbesondere der Metabolite, meist störend. Daher sind der Zelllyse nachfolgende Reinigungsschritte wie insbesondere chromatographische und/oder elektrophoretische Auftrennungen erforderlich, um die Inhaltsstoffe, insbesondere die Metabolite, in zweckmäßiger Reinheit zu erhalten. Dies ist insbesondere für die weitere Verarbeitung beziehungsweise zum Nachweis oder Analyse erforderlich, um beispielsweise eine spezifische Analyse der Metabolite mittels chromatographischer, enzymatischer beziehungsweise massenspektroskopischer Methoden zu ermöglichen. Ein verbessertes Verfahren zur Gewinnung von Inhaltsstoffen, insbesondere von Metaboliten, aus biologischen Zellen, welches insbesondere einfach durchzuführen und die Kontamination der freigesetzten Inhaltsstoffe mit störenden Komponenten wie Zelltrümmer, nackte DNA und Proteinkomplexe minimiert ist, ist daher wünschenswert.

Weiter sind Probennahmeverfahren, insbesondere zum Überführen von Proben biologischer Zellen aus Kultivierungsgefäßen, beispielsweise einem Bioreaktor, in ein Probengefäß bekannt. Solche Probennahmeverfahren dienen in erster Linie zur Erforschung physiologischer Parameter biologischer Zellen und zur Analyse der Prozessführung in der Biotechnologie und der Bioverfahrenstechnik. Folgende Parameter sind dabei erstrebenswert: (1) eine möglichst schnelle Überführung von Zellsuspension aus einem Zellkultivierungssystem in ein Probennahmegefäß, um gegebenenfalls eine hohe Probennahmefrequenz zu ermöglichen ("Transfer"); (2) die prompte Abstoppung des Metabolismus, damit die interessierenden Metabolite nicht durch enzymatische Reaktionen weiter umgesetzt werden ("Quenching"); (3) die möglichst vollständige Freisetzung der Metabolite aus den Zellen durch Anwendung verschiedener Aufschlussverfahren ("Aufschluss"); (4) die in der Probenlösung enthaltenen Metabolite müssen von Zelltrümmern und nicht lysierten Zellen getrennt werden ("Abtrennung"); optional müssen (5) für eine quantitative Biologie weitere Randbedingungen wie die Berücksichtigung der Stabilität von Metaboliten in den durchlaufenen Schritten sowie deren Freisetzungsgrade beziehungsweise deren Adsorptionsverhalten an Komponenten der Zellsuspension und Hilfsmaterialen eingehalten werden ("Quantifizierbarkeit").

Ein schnelles Probennahmeverfahren wurde von Theobald und Mitarbeitern (In vivo analysis of glucose-induced fast changes in yeast adenine nucleotide pool applying a rapid sampling technique. Anal Biochem. (1993) 214:31-7) entwickelt. Diese Probennahme besteht aus einer dünnen Edelstahlkapillare (Innendurchmesser 0,7 mm) und einem Kugelhahn. Konstruktive Maßgabe war die Minimierung des Totvolumens (200 $\mu$l), da die Probennahme durch diskretes Öffnen/Schließen des Hahns erfolgt. Bei einem Gesamtvolumen von ca. 5 ml je Probe ist das Totvolumen vernachlässigbar klein. Diese Probennahme integriert weder Schritte zum Abstoppen des Metabolismus noch zur Metabolitextraktion. Auch sind diskrete Öffnungs-/Schließvorgänge des Kugelhahns ohne jede Automatisierung vorgesehen. Die Zellsuspension wird durch vakuumierte Proberöhrchen gefördert (Vakuumpumpe, 8x10$^{-3}$ mbar), die Probennahmefrequenz beträgt, da manuell, nur ca. 0,2 s$^{-1}$.

Eine Weiterentwicklung ist die von Lange und Mitarbeitern (Improved rapid sampling for in vivo kinetics of intracellular metabolites in Saccharomyces cerevisiae. Biotechnol. Bioeng. (2001) 75:406-15.) verwendete Probennahme. Eine Reduzierung des effektiven Totraumvolumens auf 50 $\mu$l pro Probennahme wird durch den Einbau einer Bypassleitung und Ansteuerung je eines Ventils für die Probennahme- bzw. die Bypassleitung erreicht. Durch schnelle Ventilumschaltung zwischen diesen beiden Leitungen kann so verhindert werden, dass Probe aus dem Totraum gezogen wird. Außerdem wird der Verlust an Probe minimiert. Die geometrischen Abmessungen (Innendurchmesser der Kapillare: 1,0 mm, Länge: 80 mm) sind vergleichbar der Probennahme von Theobald. Die Förderung der Zellsuspension erfolgt ebenfalls diskret durch ein Vakuum in den versiegelten Reagenzgläsern (Vakuumpumpe). Die Probennahmefrequenz ist ebenfalls niedrig und beträgt ca. 1,5 s$^{-1}$ allein für die Fluidförderung. Dieser Wert für die Probennahmefrequenz berücksichtigt allerdings nicht die Zeit für die Ausbildung des Vakuums (ca. 1 s) sowie die für den manuellen Wechsel der einzelnen Reagenzgläser benötigte Zeit.

In den bekannten Probennahmevorrichtungen findet sowohl das Abstoppen des Metabolismus als auch die Metabolitextraktion in konstruktionstechnisch getrennten Einheiten statt, und damit zeitlich getrennt und nicht integriert während der Probennahme und durch das Probennahmesystem selbst. Dabei sind Quenching und Metabolitfreisetzung teilweise getrennte Prozessschritte, teilweise aber auch durch chemische Einwirkungen einer zweiten Phase (z. B. Säuren, Basen, flüssigen Stickstoff, tiefgekühltes Methanol) in einem Schritt zusammengefasste Operationen.

Aus der DE 4407439 A1 ist ein "Verfahren und Vorrichtung zur Serienprobennahme biologischer Proben" bekannt, worin halb-kontinuierlich eine Probe in eine Polypropylen-Rohrwendel (Innendurchmesser: 8 mm, Länge: 100 m, Wendeldurchmesser: 0,5 m) gezogen wird bis diese mit Zellsuspension und parallel zudosierter Perchlorsäure (35% w/v, bei -25°C) gefüllt ist. Durch die Perchlorsäure erfolgt eine sofortige Inaktivierung des Metabolismus bei gleichzeitiger Zerstörung der Zellstruktur und Freisetzung der Metabolite. Wenn die Rohrwendel gefüllt ist, wird diese bei -80°C gefroren. Diese kann dann in Stücke zerteilt aufgetaut werden, wobei sich die Lage eines Probesegments im Schlauch zum Beispiel mit einem bestimmten Zeitpunkt der Änderung der Kultivierungsbedingung, wie der dynamischen Anregung (z.B. pulsartige Erhöhung der Glucosekonzentration in glucoselimitierter Kultur), korrelieren lässt.

Eine kontinuierliche Probennahme wird in der DE 19705289 A1 beschrieben. Die Probennahme erfolgt kontinuierlich, ein gleichzeitiges Quenchen sowie die Freisetzung von Metaboliten dadurch ist aber nicht vorgesehen.

Somit sind die wesentlichen Grundanforderungen an jedes Quenching-, Aufschluss- und Abtrennungsverfahren (i.) ein möglichst schnelles Abstoppen des Metabolismus, das heißt vollständige, irreversible Inaktivierung/ Denaturierung der Enzyme beziehungsweise deren sofortige Abtrennung, (ii.) eine reproduzierbare, daher in der Regel möglichst vollständige Zerstörung der Zellstruktur bzw. Permeabilisierung der Zellmembran zur vollständigen Freisetzung der Metabolite aus den Zellen, (iii.) eine leichte Abtrennung von Metaboliten, Zellteilen und Zellen. Weiter dürfen (iv.) die zu untersuchenden Metabolite durch die Maßnahmen (i.) bis (iv.) in ihrer strukturellen Integrität (chemischen Struktur) nicht dermaßen beeinflusst werden, dass keine eindeutige Identifikation und Analyse mehr möglich ist. Ferner muss für eine quantitative Analyse in den einzeinen Schritten eine reproduzierbare Freisetzung und eine negative Beeinflussung der Messung durch ein nicht reproduzierbares Adsorptionsverhalten der Metabolite an Hilfsmitteln vermieden werden.

Typischerweise umfassen die Verfahrensschritte Quenching/Aufschluss/Abtrennung Zwei-Phasen-Systeme zum Quenching und Aufschluss, Filtration und Zentrifugation zur Abtrennung. Ein Abstoppen des Metabolismus kann zum einen durch chemische Reagenzien erfolgen, zum anderen durch Anwendung eines Temperaturgradienten.

Nach Theobald et al. (s.o.) wird in versiegelte Reagenzgläser unter Vakuum Perchlorsäure (35% w/v bei -20°C) vorgelegt, nutzt also Temperaturen unterhalb des Gefrierpunktes wässriger Lösungen zum Abstoppen des Metabolismus bei überlagerter Extraktion mittels gekühlter Perchlorsäure. Weiterhin wurden drei Gefrier-Tauzyklen durchgeführt (durch Eiskristallbildung wird die Zellwand dabei permeabilisiert). Vor der Analyse müssen die Proben neutralisiert werden, wobei der sich bildende KClO$_4$-Niederschlag in einem Filtrationsschritt beseitigt wird.

Nach Lange et al. (s.o.) werden ebenfalls Temperaturen unterhalb des Gefrierpunktes wässriger Lösungen eingesetzt, wobei 5 ml Methanol (-40°C) als Quenchingmittel vorgelegt werden. Die Metabolitextraktion erfolgt in dem durch Zentrifugation erhaltenen Zellpellet durch kochendes Ethanol.

Gemäß DE 19705289 A1 wird eine Methanolmischung bei -50°C als Quenchingflüssigkeit mit anschließender Zentrifugation (-20°C), Resuspension des mit Perchlorsäure und einem Gefrier-Tauzyklus (-80°C) zur Metabolitextraktion, Neutralisierung der Lösung und erneuter Zentrifugation zur Entfernung des Niederschlags eingesetzt.

Weiter werden flüssiger Stickstoff in Verbindung mit Gefrier-Tauzyklen zum Quenchen beziehungsweise Extrahieren verwendet sowie anstelle von kochendem Ethanol auch kochendes Wasser (Bhattacharya, Fuhrman, Ingram, Nickerson, Conway. Single-run separation and detection of multiple metabolic intermediates by anion-exchange high-performance liquid chromatography and application to cell pool extracts prepared from Escherichia coli. Anal. Biochem. (1995) 232:98-106).

Weiter sind Vorrichtungen bekannt, die dem Bauprinzip eines Wärmetauschers entsprechen. Sie werden insbesondere als Sterilisationseinheiten zur thermischen Behandlung kleiner Volumenströme eingesetzt. Aus der EP 0722075 A1 ist eine kontinuierlich zu betreibende Sterilisationseinheit für den Labor-/Technikumsmaßstab bekannt. Dabei wird der Gesamtvolumenstrom in mehrere gleichlange Rohre über aufwändig gestaltete Verteilerblenden symmetrisch aufgeteilt. Zur Sterilisation ist die Kurzzeiterhitzung bei ca. 140°C vorgesehen.

Ein dampfbeheizter Wärmeaustauscher ist aus der DE 2345243 A1 bekannt. Charakteristisch ist der optimierte Wärmeübergang durch wendelförmige Strömungsführung und geometrische Veränderung der Strömungsquerschnitte. Aus der WO 92/16807 ist eine Vorrichtung bekannt, die ebenfalls zur Vergrößerung der Oberfläche und Optimierung der Strömungsverhältnisse Rohrwendeln verwendet und als Besonderheit Flüssigkeitsinjektoren aufweist. Diese sorgen für zusätzliche Verwirbelung und damit einen verbesserten Wärmeübergang.

Die Kurzzeiterhitzung biologischer Proben durch Mikrowellen ist ebenfalls bekannt, beispielsweise aus der EP 0217662 A1. Beschrieben sind sowohl das Verfahren der Sterilisation mittels Mikrowellen, als auch die hierfür erforderliche Vorrichtung.

Die US 6,197,553 B1 beschreibt Verfahren zur Thermolyse von Bakterienzellen mittels Kapillare und Wärmetauscher. Dabei wird Zellsuspension durch eine beheizte Kapillare gepumpt. Dabei wird so viel Wärme übertragen, dass es zu einer Lyse der Zellen kommt und Plasmid-DNA aus den Zellen freigesetzt wird. Anschließend muss die Plasmid-DNA aus dem entstandenen Zell-Lysat isoliert und aufgereinigt werden.

Keine dieser bekannten Vorrichtungen ist eine Zellkonditioniereinrichtung und wird für die Freisetzung und Isolierung von Inhaltsstoffen, insbesondere Metabolite, biologischer Zellen eingesetzt.

Ausgehend vom Stand der Technik liegt der vorliegenden Erfindung das technische Problem zugrunde, Verfahren für die Konditionierung biologischer Zellen sowie Mittel dazu bereitzustellen, die im Wesentlichen die verbesserte Freisetzung und Isolierung von Inhaltsstoffen aus der biologischen Zelle und insbesondere einen verbesserten quantitativen und qualitativen Nachweis der freigesetzten Inhaltsstoffe ermöglicht.

Das technische Problem wird gelöst durch die Bereitstellung eines Verfahrens zum thermischen Konditionieren einer biologischen Zelle, wobei die Zelle bei einer Kultivierungstemperatur $T_M$ in einem Kulturmedium kultiviert wird und anschließend für eine Konditionierzeit $t_h$ bei einer Konditioniertemperatur $T_K$ konditioniert wird, welches dadurch gekennzeichnet ist, dass dabei das aus der Kultivierungstemperatur $T_M$, Konditioniertemperatur $T_K$ und Konditionierzeit $t_h$ gemäß der Formel

$$WE = t_h \cdot (T_K - T_M)$$

errechnete Wärmeäquivalent **WE**, Werte von 90 K·s bis 150 K·s annimmt.

Das heißt, die Parameter Kultivierungstemperatur $T_M$, Konditioniertemperatur $T_K$ und Konditionierzeit $t_h$ werden gemäß der Erfindung stets so gewählt, dass das errechnete Wärmeäquivalent **WE** von 90 K·s bis 150 K·s beträgt, wobei die Konditioniertemperatur $T_K$ von 80°C bis 95°C beträgt.

Ein wesentlicher Aspekt der vorliegenden Erfindung ist die Wärmebehandlung von Zellen zur vollständigen Freisetzung von Metaboliten ohne die Zerstörung der strukturellen Integrität der Zellen. Strukturelle Integrität der Zelle bedeutet die Beibehaltung der Hülle der Zelle in einem Stück und keine Bildung von Bruchstücken daraus. Damit findet auch keine signifikante Bildung von Zellfragmenten statt (subzelluläre Partikel wie Micellen aus der Cytoplasmamembran, nackte DNA-Bruchstücke, mRNA, Proteinkomplexe). Durch das einfache Durchlichtmikroskop bei einer 500fachen Gesamtvergrößerung (Okular x Tubus x Objektiv = 10 x 1.25 x 40) betrachtet, sind beispielsweise vollständig intakte E. coli-Zellen und E. coli-Zellen mit struktureller Integrität in ihrer Größe nicht unterscheidbar,

Die Erfinder fanden überraschend, dass durch die erfindungsgemäße thermische Konditionierung in einem durch das Wärmeäquivalent **WE** vorgegebenen engen Temperaturbereich und dem genau darauf abgestimmten Zeitintervall die Freisetzung der zellulären Inhaltsstoffe, insbesondere der Metabolite, erreicht wird, wobei gleichzeitig, besonders vorteilhaft, die Zellstruktur der thermisch konditionierten biologischen Zelle erhalten bleibt. Im Wesentlichen liegen nach dem erfindungsgemäßen thermischen Konditionieren neben den freigesetzten Inhaltsstoffen, insbesondere den Metaboliten, "entleerte" Zellhüllen mit intakter Struktur vor. Das Auftreten von Zelltrümmern und weiteren störenden Komponenten ist vermindert und wird bei zweckmäßiger Wahl der Parameter und je nach Anwendungsgebiet sogar ganz verhindert. Besonders vorteilhaft ist eine einfache und effiziente Abtrennung dieser Zellhüllen mit intakter Struktur von den Metaboliten mittels einfacher Filtration und/oder Zentrifugation möglich. Dadurch wiederum wird, besonders vorteilhaft, eine sich insbesondere ummittelbar an die Konditionierung anschließende quantitative und/oder qualitative Analyse der intrazellulären Metabolite, ohne weitere aufwändige Zwischenschritte, ermöglicht.

Anwendungsgebiete der erfindungsgemäßen technischen Lehre sind insbesondere Verfahren und Vorrichtungen zur Bestimmung intrazellulärer Metabolitkonzentrationen in verschiedenen Zellsystemen wie Mikroorganismen, Pilzen oder Zellkulturen tierischer, menschlicher, pflanzlicher Zellen bei Züchtung in Kultivierungssystemen wie Bioreaktor, Schüttelkolben, Proberöhrchen oder Microtiterplatten.

Untersucht werden können gemäß der Erfindung sowohl stationäre als auch nicht stationäre physiologische Zustände der biologischen Zelle(**n**). Der Metabolismus kann dabei schnellstmöglich abgestoppt werden. Die selektive Metabolitfreisetzung durch die erfindungsgemäß thermisch konditionierten Zellen ermöglicht die einfache Probenaufarbeitung und Aufreinigung für die Analyse.

So wird beispielsweise bei der Durchführung enzymatischer Assays und der Bestimmung der Lichtstreuung und absorption im Photometer die bei bekannten Freisetzungsverfahren durch das Ausfallen bestimmter Zelltrümmer und anderer störender Komponenten im Medium unvermeidliche Trübung der Probenlösung, wie sie bei der vollständigen Zerstörung der Zellstruktur und der subsequenten Abtrennung durch Filtration beobachtet wird, vermieden. Bei chromatographischen Methoden, beispielsweise bei der HPLC-Analyse, wird die Blockade der HPLC-Säule durch große Zelltrümmer vermieden.

Insbesondere werden die vorteilhaften Effekte bei einem Temperaturbereich von 80°C bis 95°C erreicht, jedoch wird auch bei davon abweichenden Werten der Konditioniertemperatur nach oben oder nach unten, je nach Zweck der Anwendung und Anwendungsgebiet ein vorteilhafter Effekt erreicht. Die im Einzelfall einzusetzende Konditioniertemperatur wird der Fachmann aufgrund seines Fachwissens leicht finden können. In einer bevorzugten Variante liegt die Konditioniertemperatur $T_K$ stets unter dem Siedepunkt des Kulturmediums. Gemäß der Erfindung wird bevorzugt - und im Gegensatz zum Stand der Technik - ein Sieden des die biologischen Zellen enthaltenden Kulturmediums stets vermieden.

In einer bevorzugten Ausführung des erfindungsgemäßen Verfahrens beträgt die Konditionierzeit $t_h$ von 0,5 s bis 600 s, bevorzugt von 1 s bis 180 s. Auch hier werden die vorteilhaften Effekte insbesondere bei einer Konditionierzeit $t_h$ von 0,5 s bis 600 s erreicht. Jedoch wird auch bei davon abweichenden Werten der Konditionierzeit nach oben oder nach unten, je nach Zweck der Anwendung und Anwendungsgebiet eine vorteilhafter Effekt erreicht. Die im Einzelfall einzusetzende Konditionierzeit wird der Fachmann aufgrund seines Fachwissens leicht finden können.

In einer bevorzugten Ausführung des erfindungsgemäßen Verfahrens beträgt die Kultivierungstemperatur $T_M$ von 26°C bis 42°C, bevorzugt von 30°C bis 38°C. Die im Einzelfall einzusetzende Kultivierungstemperatur $T_M$ der biologischen Zellen wird der Fachmann aufgrund seines Fachwissens leicht finden können.

Ein weiterer bevorzugter Gegenstand ist eine Variante des erfindungsgemäßen Verfahrens, welches zur thermischen Konditionierung eines gram-negativen Prokaryonten als biologische Zelle eingesetzt wird, wobei das für diesen Organismus optimale Wärmeäquivalent **WE** $110 \pm 20$ K·s beträgt. Ein Beispiel für den gram-negativen Prokaryonten ist das Bakterium Escherichia coli (E. coli). Ein weiterer bevorzugter Gegenstand ist eine Variante des erfindungsgemäßen Verfahrens, welches zur thermischen Konditionierung eines Eukaryonten als biologische Zelle eingesetzt wird, wobei das für diesen Organismus optimale Wärmeäquivalent **WE** $110 \pm 20$ K· s beträgt. Ein Beispiel für den Eukaryonten ist der Pilz Saccharomyces cerevisiae (S. cerevisiae).

Ein weiterer bevorzugter Gegenstand ist eine Variante des erfindungsgemäßen Verfahrens, welches zur thermischen Konditionierung eines gram-positiven Prokaryonten als biologische Zelle eingesetzt wird, wobei das für diesen Organismus optimale Wärmeäquivalent **WE** $130 \pm 20$ K· s beträgt. Ein Beispiel für den gram-positiven Prokaryonten ist das Bakterium Bacillus subtilis (B. subtilis).

Ein weiterer bevorzugter Gegenstand ist eine Variante des erfindungsgemäßen Verfahrens, wobei das Kulturmedium ein flüssiges Medium ist und das thermische Konditionieren dadurch erfolgt, dass das die biologische Zelle enthaltende flüssige Medium in einer Kapillare fließt, wobei sich die Zelle für eine Konditionierzeit $t_h$ innerhalb einer auf die Konditioniertemperatur $T_K$ temperierten Kapillarstrecke der Kapillare befindet. So wird erfindungsgemäß bevorzugt das entsprechende zweckmäßige Wärmeäquivalent übertragen und die biologischen Zellen gemäß der Erfindung thermisch *konditioniert. In einer bevorzugten Variante beträgt der Volumenstrom in der temperierten Kapillarstrecke von 0,5 ml/s bis 12 ml/s. Besonders bevorzugt beträgt der Volumenstrom von 2,5 ml/s bis 8,0 ml/s.

Ein weiterer bevorzugter Gegenstand ist eine Variante des erfindungsgemäßen Verfahrens, wobei der Schritt der thermischen Konditionierung mit der Überführung des die biologischen Zellen enthaltenden Kulturmediums aus einem Kultivierungsgefäß, insbesondere einem Bioreaktor, in ein Auffanggefäß, insbesondere ein Probensammelgefäß, durchgeführt wird. Dadurch wird vorteilhaft eine schnelle Überführung von beispielsweise Zellsuspension aus einem Zellkultivierungssystem in ein Probennahmegefäß, die prompte Abstoppung des Metabolismus in der Zelle und gleichzeitig die Freisetzung der Metabolite aus den Zellen in einem einzigen Verfahrensschritt der an die Probenüberführung gekoppelten thermischen Konditionierung der biologischen Zellen erreicht. Besonders vorteilhaft wird so gemäß der Erfindung eine sich an diese Probenüberführung unmittelbar anschließende quantitative und/oder qualitative Analyse der freigesetzten oder des speziellen interessierenden Metaboliten sowie deren oder dessen Isolierung und Gewinnung ermöglicht. Insbesondere steht der Metabolit nach der an die Probenüberführung gekoppelten thermischen Konditionierung bereits nach einfacher Filtration oder Zentrifugation in zweckmäßig hoher Reinheit zur Verfügung. Auf aufwändige Reinigungsschritte kann verzichtet werden, so dass sowohl eine zeitnahe kontinuierliche Analyse beziehungsweise Gewinnung der Metabolite, als auch eine hohe Probennahmefrequenz realisiert werden kann. Außerdem kann ein hoher Probendurchsatz in gleich bleibender, von wenigen Faktoren abhängiger Qualität erzielt werden.

Die vorliegende Erfindung betrifft auch die Gewinnung sowie die quantitative und qualitative Analyse beziehungsweise den quantitativen und qualitativen Nachweis der in der biologischen Zelle enthaltenen Inhaltsstoffe, insbesondere mindestens eines intrazellulären Metaboliten. Dieses ist vorzugsweise ausgewählt aus der Gruppe der Metaboliten, bestehend aus Aminosäuren, Aminen und deren Derivate, Carboxylsäuren, Alkoholen, Aldehyden, Ketonen, Phosphatestern (ohne die Nukleinsäuren), Nukleinsäuren und verwandten Verbindungen, Zuckern und verwandten Verbindungen, Lipiden, Steroiden, Fettsäuren, Vitaminen, Coenzymen und anorganischen Ionen.

Die mittels der nachstehend beschriebenen erfindungsgemäßen Verfahren gewonnenen, quantitativ bestimmten beziehungsweise qualitativ nachgewiesenen Metabolite sind insbesondere:

| Metaboliten-Klasse: | konkrete Metabolite: |
| --- | --- |
| Aminosäuren, Amine und deren Derivate | Alanin, Glutamat |
| Carboxylsäure | Pyruvat, Citrat, Isocitrat, Oxoglutarat, Oxaloacetat, Malat, Fumarät, Succinat, Phosphoglycerat, Phosphoenolpyruvat |
| Alkohole | Ethanol |
| Aldehyde | Glycerinaldehydphosphat |
| Phosphatester (ohne Nukleinsäuren) | ppGpp, NAD, NADP, NADH, NADPH, |
| Nukleinsäuren und verwandte Verbindungen | Adenosinmonophosphat, Guanosintriphosphat, Uridindiphosphat, cAMP |
| Zucker und verwandte Verbindungen | Glucosephosphat, Fructosephosphat, Fructosebisphosphat, Phosphogluconat, Ribose- und Ribulosephosphat, Erythrosephosphat, Sedoheptulosephosphat, Dihydroxyacetonphosphat |
| Lipide, Steroide und Fettsäuren | Acetyl-CoA |
| Vitamine und Coenzyme | Riboflavin |
| Anorganische Ionen | Phosphat |

Weitere gemäß der Erfindung aus biologischen Zellen gewinnbare beziehungsweise isolierbare, quantitativ bestimmbare beziehungsweise qualitativ nachweisbare Metabolite sind: Aminobutanoat, Acetoacetat, Acetoacetyl-CoA, Acetaldehyd, Acetyl-L-carnitin, Acetyl-CoA, Acetate, Acetyladenylät, Acetohydroxybutanoat, S-Acetyldihydrolipoamide, N-Acetylglutamat, N-Acetylglutamatphosphat, N-Acetyiglutamat, N-Acetylglutamatphosphat, N-Acetylglutamatsemialdehyd, O-Acetylhomoserin, 2-Acetolactat, N2-Acetylornithin, Acetylphosphate, N-Acetylglucosamin, N-Acetylglucosaminphosphat, Acylglycerol, 1-Acyl-sn-glycerol-3-phosphat, 1-Acylglycerophosphocholin, 1-Acyl-sn-glycero-3-phosphoethanolamin, N-Acetylmannosamin, N-Acetylmannosaminphosphat, 2-Amino-3-carboxymuconatsemialdehyd, N-Acetylneuraminat, N-Acetyl-neuraminat-9-phosphat, Adenin, Adenosin, Adenylosuccinat, Adenylylsulfat, Adenylosuccinat, Adenosindiphosphat, ADP-Ribose, ADP-Ribosephosphat, 1-(5'-Phosphoribosyl)-5-formamido-4-imidazolecarboxamid, Aicar, Amino-imidazolribotid, Ketoglutarat, Ketovalin, Alanin, Aminoacetoacetat, Aminoaceton, Aminoadipat, Aminoadipatadenylat, Aminoadipatsemialdehyd, Aminobenzoat, Aminolevulinat, Adenosinmonophosphat, 2-Aminomuconat, 2-Aminomuconatsemialdehyd, Arginin, (N-Omega-L-arginino)Succinat, Arogenat, S-Adenosylmethioninamin, Asparagin, Aspartat, Asparagin, Aspartylphosphat, Aspartatsemialdehyd, Adenosintriphosphat, Betain, Betainaldehyd, CMP-N-Acetylneuraminat, cAMP, Carnitin, N-Carbamoyl-L-Aspartat, Carbamoylphosphat, Cardiolipin, Cytidindiphosphat, CDP-Cholin, CDP-Diacylglycerol, CDP-Ethanolamin, Ceramid, cGMP, Cholesterin, Cholesta-7,24-dien-3-betaol, Cholin, Cholinphosphat, Chorismat, 3-Carboxy-1-hydroxypropyl-ThPP, Aconitat, Methylaconitat, Citrat, Citrullin, Cytidinmonophosphat, Coenzym A, Coenzym Q, Kreatin, Phosphokreatin, Crotonoyl-CoA, Cytidintriphosphat, Cystein, Cystathion, Cystin, Cytidin, Deoxyadenosin, Deoxyadenosindiphosphat, Deamino-NAD, Deoxyadenosin-monophosphat, Deoxyadenosintriphosphat, Deoxycytidindiphosphat, Deoxycytidinmonophosphat, Deoxycytidintriphosphat, Cytidin, Demethyllanosterol, 4,4-Dimethyl-5-cholesta-8,14,24-trien-3-betaol, Deoxyguanosindiphosphat, Deoxyguanosinmonophosphat, Deoxyguanosintriphosphat, Deoxyguanosin, Dihydroxyacetonphosphat, Dehydrocholesterin, Dihydrofolat, Dihydrolipoamid, Dihydroneopterin, (S)-Dihydroorotat, Dihydropteroat, Dihydrobiopterin, Amino-4-hydroxy-6-hydroxymethyl-dihydropteridin, 2-Amino-7.8-dihydro-4-hydroxy-6-(diphosphooxymethyl)pteridin, 33-Dehydroquina, Dehydrosphinganin, Diacylglycerol, Diacylglycerolphosphat, Dimethylglycin, Deoxyinosin, Dimethylallyldiphosphat, Dimethylglycin, N-Acetyl-D-glucosaminyldiphosphodolichol, N,N'-Chitobiosyldiphosphodolichol, Dolichol, Dolichylphosphat, Dolichyldiphosphat, Dehydropantoat, Dephospho-CoA, 2-Deoxy-D-ribosephosphat, 3-Dehydroshikimat, Deoxythymidindi-phosphat, Deoxythymidinmonophosphat, Deoxythymidintriphosphat, Deoxyuridindiphosphat, Deoxyuridinmonophos-phat, Deoxyuridin, Deoxyuridintriphosphat, Erythrosephosphat, Ethanolamin, Ethanol, Fructosephosphat, Fructosebisphosphat, FAD, Farnesyldiphosphat, 2-(Formamido)-N1-(5'-phosphoribosyl)acetamidin, 5'-Phosphoribosyl-N-formylglycinamid, Fumarylacetoacetat, Formiminotetrahydrofolat, Formylkynurenin, Fosäure, Formiat, Formaldehyd, Formiminogglutama, Fructose, Formyltetrahydrofolat, Formyltetrahydrofolat, Fumarat, Glucosephosphat, Glycerat-2-phosphat, Glycerat-3-phosphat, Galactose, alpha-DGalactose-1-phosphat, D-Glyceraldehyd, Glyceraldehyd-3-phosphat, 5-Phosphoribosylglycinamid, Guanosindiphosphat, GDP-Dehydrodeoxymannose, GDP- Fucose, GDP-Mannose, Geranyldiphosphat, Gluconolactonphosphat, Glyceraldehyd, Glucosamin, Glucosaminphosphat, Glucose, Glutamin, Glycolat, Glycerophosphocholin, Glycerophosphoethanolamin, Gluconat, Phosphogluconat, Glutamat, Glutamatphosphat, Glutamatsemialdehyd, Glutamyl-L-cystein, Glutathion, Glutaryl-CoA, Glycin, Glycerol, Glycerolphosphat, Glycolaldehyd, Glyceron, Glyoxylat, Glycerat, Guanosinmonophosphat, Guanosintriphosphat, Guanin, Guanosin, Guanidinoacetat, Wasserstoffionen, Wasserstoffperoxid, Hydroxyanthranilat, Hydroxybutanttricarboxyla, Homocitrat, Hydrogencarbonat, Homocystein, Hydroxykynurenin, Hydroxyprolin, Hydroxypyruvat, Histidin, Homoisocitrat, Hydroxymethylglutaryl-CoA, Homogentisat, 3-(4-Hydroxyphenyl)pyruvat, Sulfid, Homoserin, O-Phosphohomoserin, 2-(alpha-Hydroxyethyl)thiamindiphosphat, Hypoxanthin, Hydroxybutanoyl-CoA, Hydroxyisobutyryl-CoA, Hydroxyisobutyrat, (2S,3S)-3-Hydroxy-2-methylbutanoyl-CoA, 4-Hydroxyphenylpyruvat, Isoleucin, 4-Imidazolon-5-.propanoat, D-erythro-1-(Imidazol-4-yl)glycerol-3-phosphat, Inosinmonophosphat, Indol, Indolglycerolphosphat, Inosin, Isobutyryl-CoA, Isocitrat, Isopropylmalat, Isopentenyldiphosphat, Kaliumion, Kynurenin, Lactose, Lactat, Lactaldehyd, Lanosterol, Lathosterol, Leucin, Lactoylglutathion, Lipoamid, Linolensäure, Linolsäure, Lysin, Malat, Mallonyl-Co, Mannose, Mannosephosphat, Methenyltetrahydrofolat, Methionin, Methylacetoacetyl-CoA, Methylbutenoyl-CoA, Methyloxopentanoat, Methylacrylyl-CoA, Methylthioadenosin, Methylglyoxal, Methylglutaconyl-CoA, Methyltetrahydrofolat, Methanethiöl, Methylmalonatsemialdehyd, Methylentetrahydrofolat, Mevalonat, Phosphomevalonat, Diphosphomevalonat, Inositol, Maleylacetoacetat, Mercaptopyruvat, Methyltetrahydropteroyltriglutamat, Natriumion, NAD, NADH, Nicotinamidadenindinucleotidphosphat, Nicotinamidadenindinucleotidphosphat, Ammoniumion, Nicotinat, Nicotinamid, Nicotinamidribonucleotid, Nicotinatribonucleotid, Oxaloacetat, Hydroxidion, Oleinsäure, Oleoyl-CoA, Ornithin, Orotat, Orotidinphosphat, Oxoadipat, Methyloxobutanoat, Oxobutanoat, Oxalosuccinat, Orthophosphat, Phosphoadenylylsulfat, Palmitat, Palmitoyl-CoA, Palmitionat, Pantothenat, Pantoat, Pantethein, Phosphoenolpyruvat, Phosphoethanolamin, Bisphosphoglycerat, Phenylalanin, Phenylpyruvat, Phosphohydroxypyruvat, Pyrophosphat, Phosphopantothenat, Pantetheinphosphat, Phosphopantothenoylcystein, Prephenat, Phosphoribosylglycinamid, Phosphoribosylamin, Prolin, Propanoat, Propanoyl-CoA, Phosphoribose-1-diphosphate, Phosphoserin, Presqualendiphosphat, Phosphatidylcholin, Phosphatidyl-N-dimethylethanolamin, Phosphatidyl-ethanolamin, Phosphatidylglycerol, Phosphatidylglycerophosphat, Phosphatidyl-N-methylethanolamin, 1-Phosphatidyl-D-myo-inositol, Phosphatidylserin, Putrescin, Pyrrolincarboxylat, Pyruvat, Pyridoxalphosphat, ppGpp, Quinolinat, 1-(5-Phospho-D-ribosyl)-5-amino-4-imidazolcarboxylat, Ribose, Ribosephosphat, Ribulosephosphat, Methyl-3-oxopropanoyl-CoA, Thioschwefelsäureion , Adenosylhomocystein, 1-(5'-Phosphoribosyl)-5-amino-4-(N-succinocarboxamid)-imidazol, Adenosylmethionin, Saccharopin, Sorbitol, Sedoheptulose-7-phosphat, Serin, Methyl-3-oxopropanoyl-CoA, Sulfition, Sulfation, Spermidin, Sphingomyelin, Sphinganin, Sphingosin, Squalen, Squalenepoxid, Stearat, Stearoyl-CoA, Succinat, Succinyl-coA, S-succinyl-dihydrolipoamid, Succinylhomoserin, Succinatsemialdehyd, Thiocystein, Tetrahydrofolat, Tetrahydrobiopterin, Thiamindiphosphat, Threonin, Thymidin, Tetrahydropteroyltriglutamat, Trehalose, Trehalosephosphat, Triacylglycerol, Tryptophan, Tyrosin, UDP-N-acetyl-D-glucosamin, UDP-N-acetyl-D-galactosamin, Ubiquinol, Ubiquinon, Uridindiphosphat, UDP-Galactose, UDP-Glucose; Uridinmonophosphat, Uracil, Harnstoff, Allophanate, Uridin, Urocanat, Uridintriphosphat, Valin, Xanthin, Xanthosin, Xanthosinphosphat, Xylulosephosphat und Zymosterol.

Demgemäß ist weiterer Gegenstand der vorliegenden Erfindung ein Verfahren zur Gewinnung eines Inhaltsstoffs, insbesondere mindestens eines Metaboliten, aus einer biologischen Zelle, enthaltend die Schritte:

a) Kultivieren mindestens einer biologischen Zelle in einem Kulturmedium,

b) thermisches Konditionieren der mindestens einen biologischen Zelle nach dem erfindungsgemäßen Verfahren, wobei beziehungsweise wodurch der Inhaltsstoff, insbesondere der mindestens eine Metabolit, aus der mindestens einen thermisch konditionierten Zelle freigesetzt wird, und

c) Isolieren des freigesetzten Inhaltsstoffs, insbesondere des mindestens einen Metaboliten, aus dem Kulturmedium.

Besonders bevorzugt wird Schritt a) des Verfahrens in einem Kulturgefäß, insbesondere in Verbindung mit flüssigem Kulturmedium, beispielsweise in einer Suspensionskultur durchgeführt. Der Fachmann kann im Rahmen der vorliegenden technischen Lehre aber auch jede andere ihm bekannte und für sein Anwendungsgebiet zweckmäßige Kulturbedingung wählen.

Besonders bevorzugt wird Schritt b) des Verfahrens in Verbindung mit der Probenentnahme und Überführung der Probe in ein Auffanggefäß, insbesondere einem Probengefäß, durchgeführt, beispielsweise in einer dafür vorgesehenen Probennahmevorrichtung.

Schritt c) des Verfahrens kann mittels bekannter Aufreinigungsverfahren in an sich bekannter Weise durchgeführt werden. Besonders bevorzugt und, gemäß der vorliegenden Erfindung besonders vorteilhaft, auch zweckmäßig und ausreichend sind einfache mechanische Trennverfahren wie die Filtration, zum Beispiel mit einem einfachen Sterilfilter, bevorzugt mit 0,2 $\mu$m Porengröße, oder die Zentrifugation. Zweckmäßigerweise dienen diese Trennverfahren der Abtrennung der im Kulturmedium vorhandenen (intakten) Zellhüllen der thermisch konditionierten Zellen. Im Permeat beziehungsweise im Zentrifugationsüberstand liegen der Inhaltsstoff, insbesondere der mindestens eine Metabolit, frei von störenden Komponenten vor. Es versteht sich, dass der Fachmann im Rahmen der vorliegenden technischen Lehre, zusätzlich oder an deren Stelle, auch jedes andere ihm bekannte und für sein Anwendungsgebiet zweckmäßiges Trenn-, Aufreinigungs- oder Isolierungsverfahren wählen kann.

Ein weiterer Gegenstand der vorliegenden Erfindung ist auch ein Verfahren zum quantitativen Nachweis eines Inhaltsstoffs, insbesondere mindestens eines Metaboliten, in mindestens einer biologischen Zelle, enthaltend die Schritte:

a) Kultivieren der Zelle in einem Kulturmedium,

b) thermisches Konditionieren der mindestens einen biologischen Zelle nach dem erfindungsgemäßen Verfahren, wobei beziehungsweise wodurch der Inhaltsstoff, insbesondere der mindestens eine Metabolit, aus der mindestens einen thermisch konditionierten Zelle freigesetzt wird, und

c) quantitativer Nachweis des freigesetzten Inhaltsstoffs, insbesondere des mindestens einen Metaboliten, im Kulturmedium.

Besonders bevorzugt wird Schritt a) des Verfahrens in einem Kulturgefäß, insbesondere in Verbindung mit flüssigem Kulturmedium, beispielsweise in einer Suspensionskultur durchgeführt. Der Fachmann kann im Rahmen der vorliegenden technischen Lehre aber auch jede andere ihm bekannte und für sein Anwendungsgebiet zweckmäßige Kulturbedingung wählen.

Besonders bevorzugt wird Schritt b) des Verfahrens in Verbindung mit der Probenentnahme und Überführung der Probe in ein Auffanggefäß, insbesondere einem Probengefäß, durchgeführt, beispielsweise in einer dafür vorgesehenen Probennahmevorrichtung.

Schritt c) des Verfahrens wird mittels bekannter quantitativer Nachweisverfahren für den interessierenden Metaboliten in an sich bekannter Weise durchgeführt.

Ein weiterer Gegenstand der vorliegenden Erfindung ist schließlich auch ein Verfahren zum qualitativen Nachweis eines Inhaltsstoffs, insbesondere mindestens eines Metaboliten, in mindestens einer biologischen Zelle, enthaltend die Schritte:

a) Kultivieren der Zelle in einem Kulturmedium,

b) thermisches Konditionieren der mindestens einen biologischen Zelle nach dem erfindungsgemäßen Verfahren, wobei beziehungsweise wodurch der Inhaltsstoff, insbesondere der mindestens eine Metabolit, aus der mindestens einen thermisch konditionierten Zelle freigesetzt wird, und

c) qualitativer Nachweis des freigesetzten Inhaltsstoffs, insbesondere des mindestens einen Metaboliten, im Kulturmedium.

Besonders bevorzugt wird Schritt a) des Verfahrens in einem Kulturgefäß, insbesondere in Verbindung mit flüssigem Kulturmedium, beispielsweise in einer Suspensionskultur durchgeführt. Der Fachmann kann im Rahmen der vorliegenden technischen Lehre aber auch jede andere ihm bekannte und für sein Anwendungsgebiet zweckmäßige Kulturbedingung wählen.

Besonders bevorzugt wird Schritt b) des Verfahrens in Verbindung mit der Probenentnahme und Überführung der Probe in ein Auffanggefäß, insbesondere einem Probengefäß, durchgeführt, beispielsweise in einer dafür vorgesehenen Probennahmevorrichtung.

Schritt c) des Verfahrens wird mittels bekannter qualitativer Nachweisverfahren für den interessierenden Metaboliten in an sich bekannter Weise durchgeführt.

Dir vorliegende Erfindung betrifft auch eine Vorrichtung zur Probennahme, insbesondere zur Überführung einer Probe biologischer Zellen aus mikrobiellen, vorzugsweise pilzlichen oder bakteriellen, Kulturen oder Mischkulturen sowie Zellkulturen pflanzlicher, tierischer oder menschlicher Zellen. Diese Zellen können in einem Kultivierungssystem wie Bioreaktor, Schüttelkolben, Proberöhrchen oder Microtiterplatten, insbesondere in einem Kulturgefäß in Form einer Suspensionskultur kultiviert werden. Die Erfindung sieht vor, dass die Probe dem Kultivierungssystem entnommen und in mindestens ein Auffanggefäß überführt wird. Dabei findet erfindungsgemäß bevorzugt die thermische Konditionierung der in der Probe enthaltenen biologischen Zelle(n) statt.

Ein weiterer Gegenstand der Erfindung ist daher eine Zellkonditioniervorrichtung als Bestandteil einer solchen Probennahmevorrichtung. Die erfindungsgemäße Zellkonditioniervorrichtung dient dem thermischen Konditionieren mindestens einer biologischen Zelle, welche vorzugsweise in einem flüssigen Kulturmedium suspendiert ist. Die erfindungsgemäße Zellkonditioniervorrichtung weist mindestens eine an einer Wärmequelle angeordnete und vom flüssigen Kulturmedium durchflossene Kapillare auf, durch die vorzugsweise die im Kulturmedium suspendierte Zellprobe überführt wird. Sie ist erfindungsgemäß dadurch gekennzeichnet, dass die Kapillare einen Innendurchmesser von 0,5 mm bis 4,5 mm, bevorzugt von 1,0 mm bis 3,0 mm, aufweist und entlang einer temperierten Kapillarstrecke von 50 cm bis 1550 cm, bevorzugt von 90 cm bis 420 cm, der Kapillare mit der Wärmequelle in Kontakt steht.

Bevorzugt dient die Zellkonditioniervorrichtung der Durchführung des vorliegend beschriebenen erfindungsgemäßen Verfahrens der thermischen Konditionierung. Wie beschrieben, zeichnet sich das erfindungsgemäße Verfahren insbesondere durch die Einhaltung bestimmter Verfahrensparameter aus, die einen überraschenden vorteilhaften technischen Effekt bewirken.

Zur Durchführung eines Probennahmeverfahrens mit eingeschlossener thermischer Konditionierung wird Zellsuspension wird aus dem Kultivierungssystem entnommen und mittels eines ersten Abschnitts der Kapillare, der als kurze Überführungsstrecke ausgelegt ist, und eines daran anschließenden zweiten Abschnitts, der als temperierte Kapillarstrecke ausgeführt ist, in ein Probennahmegefäß überführt.

Es wurde überraschend gefunden, dass diese Verfahrensparameter mit der erfindungsgemäßen Zellkonditioniervorrichtung eingehalten werden können, wenn bestimmte geometrische Rahmenbedingungen bei der Dimensionierung der Kapillare der Zellkonditioniervorrichtung eingehalten werden: Dargestellt sind in Tabelle 1 die Abhängigkeit von Rohrwendelinnendurchmesser und Länge der temperierten Kapillare für extremale Volumenströme 2,5 ml/s bzw. 8 ml/s und extremale Turnoverraten intrazellulärer Metabolite, beispielsweise ATP, von $\tau = 0,1$ s bzw. $\tau = 2,7$ s. Die gefundenen Werte lassen sich um beliebige Zwischenwerte für Volumenströme ($2.5$ ml/s $< \dot{V} < 8$ ml/s) sowie Turnoverraten ($0,1$s $< \tau < 2,7$ s) erweitern.

Tabelle 1:

| Volumenstrom [ml/s] | Turnover-rate $\tau$ [s] | WE [K s] | Rohrwendelinnendurchmesser [cm] | Länge der temperierten Kapillare [cm] |
|---|---|---|---|---|
| 2,5 | 0,1 | 70 | 0,05 | 83 |
| | | 300 | 0,05 | 1502 |
| | 2,7 | 70 | 0,2 | 92 |
| | | 300 | 0,2 | 1511 |
| 8 | 0,1 | 70 | 0,11 | 79 |
| | | 300 | 0,11 | 1498 |
| | 2,7 | 70 | 0,44 | 84 |
| | | 300 | 0,44 | 1503 |

Aufgrund der erfindungsgemäßen Geometrie der Zellkonditioniervorrichtung beziehungsweise Probennahmevorrichtung kann die optimale Metabolit-Probennahme nur in einem engen Temperaturbereich und einem genau darauf abgestimmten Zeitintervall erfolgen und dadurch die Zellsuspension dermaßen günstig beeinflusst werden, dass der Zellmetabolismus schnell gestoppt und der intrazelluläre Metabolit vollständig freigesetzt wird, die Zellstruktur aber erhalten bleibt. Die Temperatur liegt dabei stets unterhalb des Siedepunkts der Probensuspension.

Gegenstand der vorliegenden Erfindung ist daher eine Zellkonditioniervorrichtung, wobei für das erfindungsgemäß zu übertragende Wärmeäquivalent von 90 bis 150 Ks bei einer Konditioniertemperatur $T_K$ von 80°C bis 95°C eine Länge der temperierten Kapillarstrecke von 80 cm bis 1510 cm gewählt wird, wobei

a) für einen kleinen Volumenstrom von etwa 2,5 ml/s

    i. bei einer unteren Umsatzrate des Metaboliten von etwa 0,1 s der Innendurchmesser der Kapillare etwa 0,5 mm,
    ii. bei einer oberen Umsatzrate des Metaboliten von etwa 2,7 s der Innendurchmesser der Kapillare etwa 2 mm und

b) für einen großen Volumenstrom von etwa 8 ml/s

    i. bei einer unteren Umsatzrate des Metaboliten von etwa 0,1 s der Innendurchmesser der Kapillare etwa 1 mm, insbesondere 1,1 mm,
    ii. bei einer oberen Umsatzrate des Metaboliten von etwa 2,7 s der Innendurchmesser der Kapillare etwa 4 mm, insbesondere 4,4 mm

beträgt.

Durch eine entsprechende Abwandlung der Geometrie des erfindungsgemäße vorgesehenen Probennahmesystems wird beispielsweise auch eine Metabolomics-Analyse insbesondere in einem HD-screening-Verfahren möglich.

Ein weiterer bevorzugter Gegenstand ist eine Zellkonditioniervorrichtung, welche als Probennahmevorrichtung zur Entnahme einer Probe aus einer Quelle eines die Zelle enthaltenden flüssigen Kulturmediums ausgebildet ist und zusätzlich eine Probenüberführungsstrecke und eine Probensammelvorrichtung aufweist.

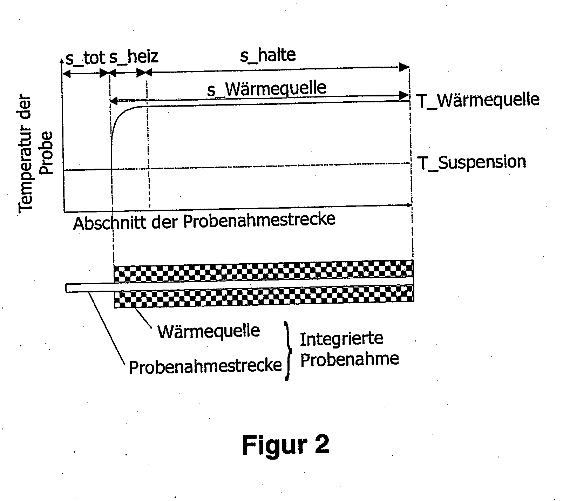

Der Temperaturverlauf der Zellsuspension in der erfindungsgemäßen Vorrichtung ist schematisch in Figur 2 dargestellt. Folgende Größen dienen der Erläuterung der erfindungsgemäßen Vorrichtung:

| | |
|---|---|
| T_Wärmequelle | = an der Kapillare angelegte Temperatur der Wärmequelle; sie entspricht der <u>Konditionier-temperatur</u> $\mathbf{T}_K$ |
| T_Suspension | = Temperatur der Suspension im Kultivierungssystem; sie entspricht der <u>Kultivierungstemperatur</u> $\mathbf{T}_M$ |
| T_Probennahmegefäß | = Temperatur der Metabolitlösung im Probennahmegefäß |
| s_tot | = Strecke vom Eingang in das integrierte Probennahmesystem bis zum Beginn der durch die Wärmequelle temperierten Kapillarstrecke |
| s_heiz | = Strecke, die für das Aufheizen der Probe vom Eintritt in die durch die Wärmequelle temperierte Kapillare bei T_Suspension bis zum Erreichen der Temperatur T_Wärmequelle durchlaufen wird |
| s_halte | = Strecke, die die Probe mit der Temperatur T_Wärmequelle in der temperierten Kapillare zurücklegt |
| s_Wärmequelle | = Länge der durch die Wärmequelle temperierten Kapillarstrecke = Summe von s_heiz und s_halte |
| t_tot | = Zeitdauer, die die Probe für das Zurücklegen von s_tot benötigt |
| t_heiz | = Zeitdauer, die die Probe für das Zurücklegen von s_heiz benötigt |
| t_halte | = Zeitdauer, die die Probe für das Zurücklegen von s_halte benötigt; sie entspricht der <u>Konditionierzeit</u> $\mathbf{t}_h$ |
| t_Wärmequelle | = Zeitdauer, die die Probe für das Zurücklegen von s_Wärmequelle benötigt |

Zieht man die Probe bei einer Temperatur der Zellsuspension T_Suspension, vorzugsweise bei Mikroorganismen und Zellkulturen typischerweise 30-37°C, aus dem Kultivierungssystem, so durchläuft diese als erstes eine Überführungsstrecke s_tot und wird beim Eintritt in die temperierte Kapillare auf einer kurzen Strecke s_heiz hinlänglich genau auf die Temperatur der Wärmequelle T_Wärmequelle gebracht, vorzugsweise +/- 5°C, um im Anschluss durch Vermischung mit bereits vorliegender Probenlösung im Probennahmegefäß auf T_Probennahmegefäß gebracht zu werden, vorzugsweise auf 4°C gekühlt. Die Länge der Aufheizstrecke s_heiz hängt dabei insbesondere von den Eigenschaften der Zellsuspension, der Geometrie des integrierten Probennahmesystems, der eingestellten Durchflussrate und der angelegten Temperatur T_Wärmequelle durch die Wärmequelle ab. Aufgrund der bevorzugt schnellen Aufheizzeiten wird zur erfindungsgemäßen Übertragung der Wärmeäquivalente die benötigte Zeit der Probe für das Durchlaufen der Aufheizstrecke s_heiz, nämlich t_heiz, insbesondere der Haltezeit t_halte bei einer Temperatur von T_Wärmequelle zugerechnet. Die Zeit t_halte bezeichnet die Zeitdauer, die ein infinitesimal kleines Volumenelement mittlerer Strömungsgeschwindigkeit für den Durchlauf der temperierten Kapillarstrecke s_halte benötigt.

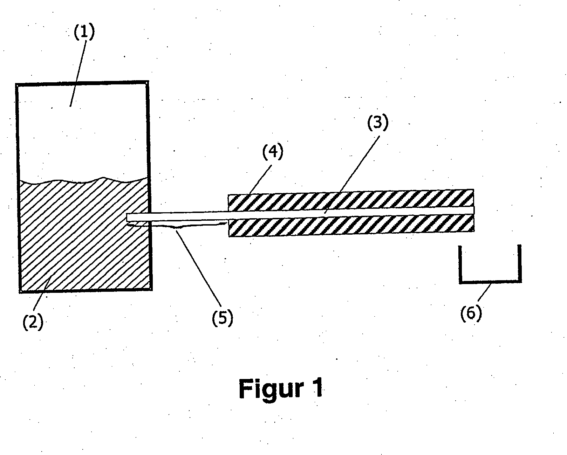

Eine zur Illustration der Erfindung beispielhaft angeführte Zellkonditioniervorrichtung beziehungsweise Probennahmevorrichtung ist in Figur 1 dargestellt. Diese weist eine Quelle (1), insbesondere einen Bioreaktor, auf, worin ein vorzugsweise flüssiges Kulturmedium (2) mit den darin enthaltenen biologischen Zellen eingefüllt ist. Vorzugsweise

liegen die Zellen in einer Suspensionskultur in dem Bioreaktor vor. Weiter weist die Vorrichtung mindestens eine Kapillare (3) auf, welche an einer Wärmequelle (4) angeordnet ist. Vorzugsweise steht die Wärmequelle über eine bestimmte Heizstrecke in einer den Wärmefluss zwischen Wärmequelle und dem Lumen der Kapillare fördernden Kopplungsweise mit der Kapillare in Verbindung; diese Heizstrecke entspricht der Länge der temperierten Kapillare.

Demgemäß ist ein weiterer Gegenstand der Erfindung die Verwendung der Zellkonditioniervorrichtung zur Gewinnung eines Inhaltsstoffs aus einer biologischen Zelle, wobei insbesondere das hierin beschriebene Verfahren dazu durchgeführt wird.

Ein weiterer Gegenstand der Erfindung ist auch die Verwendung der Zellkonditioniervorrichtung zum quantitativen Nachweis eines Inhaltsstoffs in einer biologischen Zelle, wobei insbesondere das hierin beschriebene Verfahren dazu durchgeführt wird.

Schließlich ist ein weiterer Gegenstand der Erfindung die Verwendung der Zellkonditioniervorrichtung zum qualitativen Nachweis eines Inhaltsstoffs in einer biologischen Zelle, wobei insbesondere das hierin beschriebene Verfahren dazu durchgeführt wird.

Da im folgenden die Begrifflichkeiten "Ghosts", "Ghost-Faktor", "Trues", "Trümmer", "Trümmer-Faktor" und Gesamtzellzahl eine wichtige Rolle spielen werden, sollen hier zunächst deren Definition und Bestimmungsmethoden eingeführt werden.

Im Zusammenhang mit der vorliegenden Erfindung werden unter "Ghosts" Zellen verstanden, deren Cytoplasmamembran irreversibel geschädigt wurde, die aber als vollständige "leere" Zellhüllen vorliegen. Unter "Trues" werden Zellen verstanden, deren Cytoplasmamembran intakt ist. "Trümmer" bezeichnet Bruchstücke einer Zelle, deren zelluläre Integrität zerstört wurde; hierbei handelt es sich insbesondere um Lipid-Mizellen, einzelne Proteine und mRNA, nackte beziehungsweise Protein-gebundene DNA-Bruchstücke und vieles andere mehr. Der Begriff "Ghost-Faktor" bezeichnet ein quantitatives Äquivalent, woraus die Zahl der Ghosts bestimmt wird. Der Begriff "Trümmer-Faktor" bezeichnet ein quantitatives Äquivalent, woraus die Zahl der Trümmer bestimmt wird.

Die Erfindung wird durch die nachfolgenden Figuren und Beispiele erläutert, die aber nicht beschränkend zu verstehen sind:

Es zeigen

die Figur 1    ein vereinfachtes Schema der erfindungsgemäßen Vorrichtung,

die Figur 2    ein schematisches Temperaturprofil in der erfindungsgemäßen Vorrichtung,

die Figur 3    den Grad der Metabolit-Freisetzung aus den Mikroorganismen E. coli, B. subtilis und S. cerevisiae in Abhängigkeit von der angelegten Temperatur der Wärmequelle = $T_K$. Erst bei einer Konditioniertemperatur $T_K$ über 60°C findet eine signifikante Freisetzung von Metaboliten statt. Bei einer Konditioniertemperatur $T_K$ von ca. 95°C wird eine vollständige Freisetzung erreicht. Die Konditionierzeit $t_h$ betrug 1,2 s,

die Figur 4    den Grad der Metabolit-Freisetzung bei E. coli in Abhängigkeit von der Zeit t_halte. In Abhängigkeit von der Konditioniertemperatur $T_K$ werden verschieden lange Konditionierzeiten $t_h$ für eine vollständige Metabolitfreisetzung benötigt. Dabei muss eine Schwellentemperatur T_krit,Metabolit überschritten sein,

die Figur 5    die Entstehung von Ghosts bei einer Konditioniertemperatur $T_K$ von 95°C in Abhängigkeit von der Konditionierzeit $T_h$ bei einer E. coli-Kultur,

die Figur 6    die Entstehung von Zelltrümmern durch die erfindungsgemäße Vorrichtung bei einer Konditioniertemperatur $T_K$ von 95°C in Abhängigkeit von der Konditionierzeit $t_h$ bei E. coli,

die Figur 7    die Entstehung von Ghosts in Abhängigkeit von der Konditioniertemperatur $T_K$ bei einer Konditionierzeit $T_h$ von 1,2 s bei einer Probennahme aus einer E. coli-Kultur mittels der erfindungsgemäßen Vorrichtung,

die Figur 8    den überraschenden Zusammenhang zwischen Trümmer-Bildung, Ghost-Bildung, Metabolit-Freisetzung und GZZ in Abhängigkeit von der Konditioniertemperatur $T_K$,

die Figur 9    die Abhängigkeit der Bildung von Ghosts von den angelegenten Wärmeäquivalenten. Nur in einem sehr engen Bereich von 70 bis 300 Ks WE erfolgt bei E. coli eine sehr befriedigende Bildung einer vorteilhaft hohen Zahl von Ghosts, das Optimum liegt dabei bei 110 Ks,

die Figur 10    das Ergebnis der Analyse der erfindungsgemäß bei der Probennahme und -überführung freigesetzten Metabolite ausgewählter Stoffklassen (Legende: PEP = Phosphoenolpyruvat, DHAP/GAP.= Summe von Dihydroxyacetonphosphat und Glycerinaldehyd-3-phosphat, ADP = Adenosindiphosphat, 2PG/3PG = Summe aus 2-Phosphoglycerat und 3-Phosphoglycerat, FBP = Fructosebisphosphat).

**Beispiel 1:** Freisetzung von Metaboliten

Die Fähigkeit der erfindungsgemäßen Vorrichtung, in Form eines integrierten Probennahmesystems intrazelluläre Metabolite freizusetzen, wurde exemplarisch am Beispiel der Freisetzung von ATP verfolgt. Die ATP-Konzentrationen wurden mit Hilfe eines enzymätischen Biolumineszenz-Verfahrens ermittelt (Tran & Unden Changes in the proton potential and the cellular energetics of Escherichia coli during growth by aerobic and anaerobic respiration or by fermentation Eur. J. Biochem. (1998) 251:538-43). ATP liegt vermutlich aufgrund lysierter Zellen im Medium von Zellsuspensionen wie die meisten Metabolite in der Regel nur in minimalen Mengen vor. Aufgrund seiner ionischen Phosphatgruppen kann ATP nur in äußerst vernachlässigbarer Menge über die Cytoplasmamembran diffundieren. Die Metabolitfreisetzungsversuche wurden mit E. coli-Kulturen als charakteristische Vertreter gram-negativer prokaryotischer Zellsuspensionen, mit B. subtilis-Kulturen als charakteristische Vertreter gram-positiver prokaryotischer Zellsuspensionen, und mit S. cerevisiae-Kulturen als charakteristische Vertreter eukaryotischer Zellsuspensionen durchgeführt. Eine vollständige Metabolit-Freisetzung sei im folgenden definiert als Nachweis von mehr als >95% der ATP-Menge, die bei einem schnellen Abstoppen der Zellsuspension mit kochendem Wasser für 2 Minuten und anschließendem Ultraschall-Aufschluss (30s, Ultraschallgerät, Bandelin electronic, Typ UW 2200, 12207 Berlin, Deutschland) nachgewiesen werden kann.

1.1 Freisetzung von Metaboliten: Abhängigkeit von der Konditionierzeit $t_h$

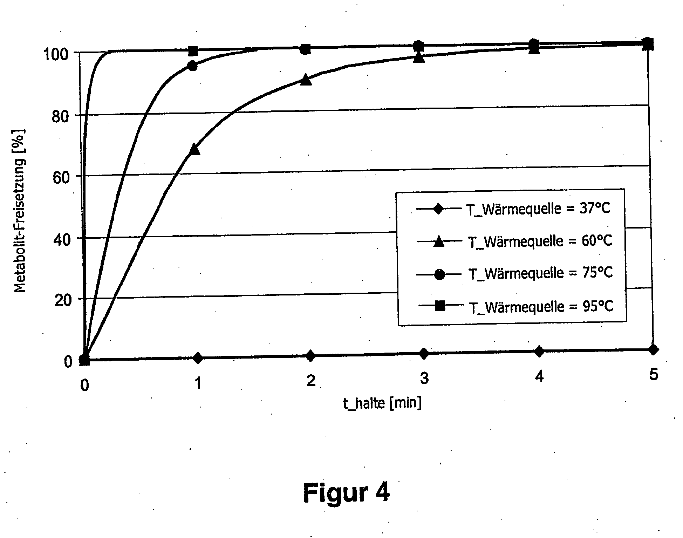

Bei einer gegebenen Temperatur T_Wärmequelle, die der Konditioniertemperatur $T_K$ entspricht, spielt die Haltezeit halte, die der Konditionierzeit $t_h$ entspricht, eine wichtige Rolle für den Grad der Metabolit-Freisetzung. Um den Einfluss der Konditionierzeit $t_h$ bei der Metabolit-Freisetzung zu untersuchen, wurde für E. coli bei verschiedenen Temperaturen (30°C, 60°C und 95°C) die Konditionierzeit $t_h$ variiert. Die Ergebnisse sind in Figur 4 dargestellt. Bei Konditioniertemperatur $T_K$= 95°C wird eine vollständige Metabolit-Freisetzung bereits bei Konditionierzeiten $t_h$ wesentlich unter einer Minute erreicht. Bei Konditioniertemperatur $T_K$ = 60°C wird im Rahmen der Versuchszeit erst bei Konditionierzeit $t_h$ = 5 min. eine vollständige Metabolitfreisetzung erreicht. Bei tieferen Temperaturen wird keine vollständige Metabolit-Freisetzung innerhalb eines akzeptablen Zeitraumes (<5 min.) mehr erreicht, bei einer Konditioniertemperatur $T_K$ von 37°C wird sogar überhaupt kein Anstieg der Metabolit-Freisetzung mehr beobachtet. Die durch die Wärmequelle angelegte Konditioniertemperatur $T_K$ muss also eine Schwellentemperatur T_krit,min übersteigen, damit im Rahmen einer vernünftigen Zeitspanne der Konditionierzeit $t_h$ von vorzugsweise weniger als 5 min. eine vollständige Freisetzung der intrazellulären Metabolite erreicht werden kann.

1.2 Freisetzung von Metaboliten: Abhängigkeit von der Konditioniertemperatur $T_K$

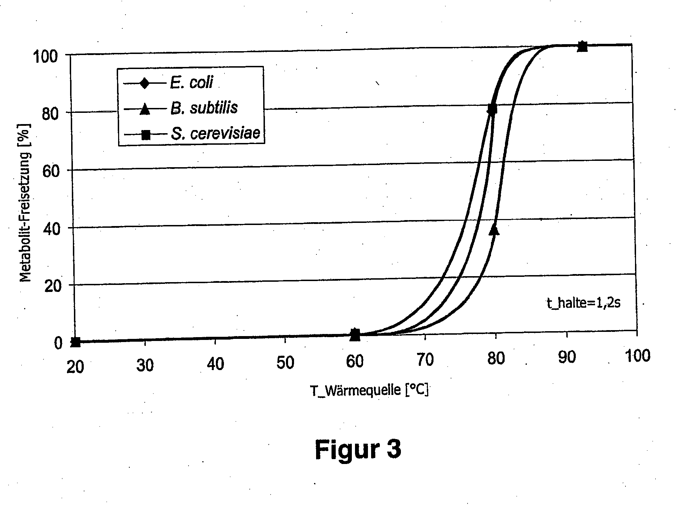

Die Abhängigkeit des Grades der Metabolit-Freisetzung von der Konditioniertemperatur $T_K$ bei einer gegebenen Konditionierzeit $t_h$ von 1,2 s wurden mit E. coli, B. subtilis- und S. cerevisiae-Kulturen durchgeführt. Die Ergebnisse sind in Figur 3 dargestellt. Während bis zu einer Konditioniertemperatur $T_K$ von 60°C keine signifikante Freisetzung von Metaboliten beobachtet werden kann, steigt der Freisetzungsgrad bei höheren Konditioniertemperaturen signifikant an und erreicht bei allen drei Zellsystemen bei spätestens 95°C eine vollständige Freisetzung der Metabolite. B. subtilis als gram-positiver Organismus zeigt gegenüber S. cerevisiae und E. coli erst bei höheren Temperaturen vergleichbare Anteile freigesetzter Metabolite.

**Beispiel 2:** Bewahrung der strukturellen Integrität

Für die Bewahrung der strukturellen Integrität von Zellen wurden im Hinblick auf das integrierte Probennahmesystem zwei entscheidende Einflussgrößen, vergleichbar mit der Metabolit-Freisetzung, identifiziert: Die Konditionierzeit $t_h$, die bei der vorzugsweise eingesetzten erfindungsgemäßen Vorrichtung der t_halte entspricht, und die angelegte Konditioniertemperatur $T_K$, die bei der vorzugsweise eingesetzten erfindungsgemäßen Vorrichtung der Temperatur T_Wärmequelle der mit der Kapillare verbundenen Wärmequelle entspricht.

Der Nachweis der "Ghosts" erfolgt zweckmäßigerweise über den Farbstoff Propidiumiodid, der aufgrund seiner positiven Ladung nur durch geschädigte Membranen permeieren kann. Propidiumiodid interkaliert in die DNA und fluoresziert daraufhin rot, weshalb auch Ghost-Zellen rot fluoreszieren. Quantitativ wurden Ghosts in der vorliegenden Patentschrift mit Hilfe des Ghost-Faktor nachgewiesen.

Der quantitative Nachweis von Ghosts erfolgt über den Ghost-Faktor. Dieser ergibt sich anhand der Formel:

Ghost-Faktor = ((rot_P-rot_Ü_P) - (rot_Ref-rot_Ü_Ref))/(grün_Ref-grün_Ü_Ref)

Hierbei ist:

rot_P = Rot-Fluoreszenz der behandelten Probensuspension

rot_Ü_P = Rot-Fluoreszenz der behandelten, abzentrifugierten Probensuspension

rot_Ref = Rot-Fluoreszenz der unbehandelten Probensuspension

rot_Ü_Ref = Rot-Fluoreszenz der unbehandelten, abzentrifugierten Probensuspension

grün_Ref = Grün-Fluoreszenz der unbehandelten Probensuspension

grün_Ü_Ref = Grün-Fluoreszenz der unbehandelten, abzentrifugierten Probensuspension

Die Rot-Fluoreszenz wurde bei 635 nm und die Grün-Fluoreszenz bei 535 nm in Mikrotiterplatten mit Hilfe eines Miktrotiterplatten-Messgerätes (TECAN GmbH, Typ Spectra Fluor, Crailsheim, Deutschland) ermittelt. Die Anregung erfolgt bei 485 nm. Bei der Messprobenvorbereitung wurde die optische Dichte der Probensuspension bei einer Wellenlänge von 600 nm bestimmt und die Probensuspension im Anschluss mittel 0,9% NaCl-Lösung auf eine Extinktion von 0,06 bei 600 nm verdünnt. Die Probensuspension wurde darauf in zwei Aliquots aufgeteilt. Ein Aliquot wurde auf Eis gelagert und mit "Probensuspension" bezeichnet. Das zweite Aliquot wurde für 2 min. bei 14000 Umdrehungen/Minute in einer Tischzentrifuge (Eppendorf AG, Typ Centrifuge 5417R, 22331 Hamburg, Deutschland) zentrifugiert, anschließend der Überstand abgenommen. Diese Aliquot wurde als "abzentrifugierte Probensuspension" bezeichnet. Im Anschluß wurden bei Aliquots gleich weiterprozessiert. Dazu wurden 100 $\mu$l der Aliquots in die Kavität einer Mikrotiterplatte gegeben, anschließend 100 $\mu$l eines Farbstoffgemisches zugegeben. Das Farbstoffgemisch wurde aus der Komponente A des L-7007 LIVE/DEAD® BacLight™ Bacterial Viability-Kit (MoBiTec, Göttingen, Deutschland) gewonnen und beinhaltete 10 $\mu$mol/l SYTO® 9 und 10 $\mu$mol/l Propidiumiodid in bidestilliertem Wasser. Die Bezeichnung behandelt bzw. unbehandelt kennzeichnet hierbei Probensuspensionen, die entweder direkt aus dem Kultivierungssystem entnommen wurden (unbehandelt) bzw. daraufhin entweder durch das integrierte Probennahmesystem prozessiert bzw. durch alternative Methoden und Verfahren konditioniert worden waren (behandelt).

Der Nachweis der "Trues" erfolgt über den Farbstoff SYTO® 9. Dieser ist ungeladen und kann somit sowohl durch intakte als auch durch beschädigte Membranen in die DNA interkalieren. Daher markiert er, wenn er alleine verwendet wird, alle Zellen, sowohl die mit intakter (Trues) als auch die mit beschädigter (Ghosts) Membran. In Kombination mit Propidiumiodid eingesetzt (wie hier der Fall) wird SYTO® 9 durch Propidiumiodid reduziert und verdrängt. Daher fluoreszieren nur Zellen mit intakten Membranen (Trues) grün.

Der quantitative Nachweis von Trümmern erfolgt über den Trümmer-Faktor. Dieser ergibt sich anhand der Formel:

$$\text{Trümmer-Faktor} = (\text{rot\_Ü\_P} - \text{rot\_Ü\_Ref})/(\text{rot\_P} - \text{rot\_Ü\_Ref})^*(\text{rot\_P} - \text{rot\_Ref})/(\text{grün\_Ref} - \text{grün\_Ü\_Ref})$$

Hierbei ist:

rot_Ü_P = Rot-Fluoreszenz der behandelten, abzentrifugierten Zellsuspension
rot_Ü_Ref = Rot-Fluoreszenz der unbehandelten, abzentrifugierten Zellsuspension
rot_P = Rot-Fluoreszenz der behandelten Zellsuspension
rot_Ref = Rot-Fluoreszenz der unbehandelten Zellsuspension
grün_Ref = Grün-Fluoreszenz der unbehandelten Probensuspension
grün_Ü_Ref = Grün-Fluoreszen der unbehandelten, abzentrifugierten Probensuspension

Die Durchführung der Messung des Trümmer-Faktors entspricht der Vorgehensweise bei der Bestimmung des Ghost-Faktors. Der Trümmer-Faktor misst dabei hauptsächlich nackte DNA-Bruchstücke.

Die Gesamtzellzahl (GZZ) wurde mit Hilfe einer Zählkammer (Neubauer, improved) unter dem Lichtmikroskop bestimmt und entspricht der Summe aus Trues und Ghosts.

2.1 Bewahrung der strukturellen Integrität: Abhängigkeit von der Konditionierzeit $t_h$

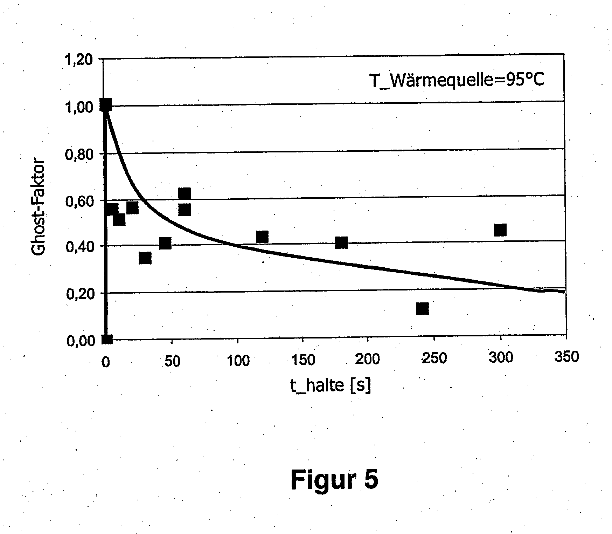

Um die Abhängigkeit der strukturellen Integrität von der Konditionierzeit $t_h$ zu untersuchen, wurden bei einer Konditioniertemperatur $T_K$ von 95°C verschiedene Konditionierzeiten $t_h$ angelegt. Die. Ergebnisse sind in Figur 5 dargestellt.

Definitionsgemäß ergibt der Ghost-Faktor bei der unbehandelten Zellsuspension einen Wert von 0. Dieser steigt bei einer Konditionierzeit $t_h$ von 1,2 s überraschenderweise sprunghaft auf 1,01 an. Er sinkt wiederum ab Konditionierzeiten $t_h$ ab 5 s schnell auf 0,56, um im weiteren Verlauf mit längerer Zeitdauer weiter stetig abzusinken, wie in Figur 5 dargestellt ist. Es kann daher geschlossen werden, dass nur eine überraschend kurze Konditionierzeit $t_h$ für die Beibehaltung der zellulären Integrität der Zellen angelegt werden muss; bei längerer Zeiteinwirkung und Temperaturen

$T_K$ über T_krit,min geht die zelluläre Integrität jedoch unerwünschterweise verloren.

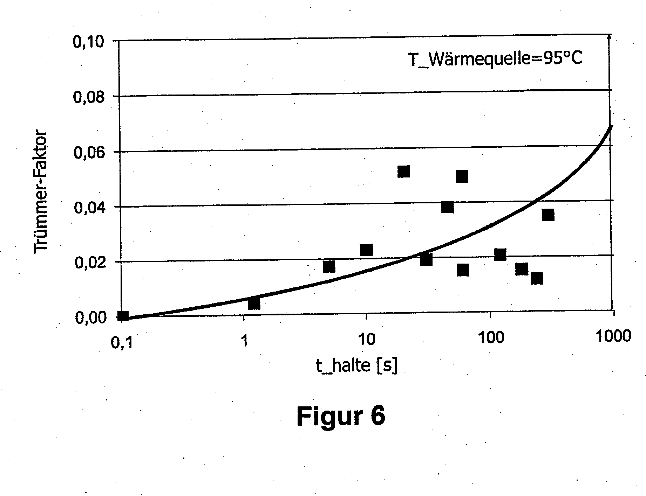

Im Gegensatz zu anderen Zellaufschlussmethoden zur Metabolit-Freisetzung, wie beispielsweise der Rührwerkskugelmühle oder dem Ultraschall, entstehen bei dem integrierten Probennahmesystem in wesentlich geringerem Ausmaß Zelltrümmer; die gefundenen Messwerte sind in Tabelle 2 dargestellt. Diese entstehen in Abhängigkeit von der angelegten Temperatur T_Wärmequelle>T_krit,min und nehmen mit der Zeit zu, siehe Figur 6.

Tabelle 2 zeigt den Trümmer-Faktor bei Probennahme und Metabolit-Freisetzung nach Behandlung der Zellsuspension mittels des integrierten Probennahmesystems, mittels Rührwerkskugelmühle und mittels Ultraschall-Aufschluss. Rührwerkskugelmühle und Ultraschall-Aufschluss zerstören die Zellintegrität und erzeugen eine wesentlich höhere Menge an Zelltrümmern als das erfindungsgemäße Verfahren.

Tabelle 2:

| Thermische Konditionierung (erfindungsgemäß) | | Trümmer-Faktor |
|---|---|---|
| Konditioniertemperatur $T_K$ [°C] | Konditionierzeit $t_h$ [s] | |
| 95 | 1,2 | 0,00 |
| 95 | 20 | 0,05 |
| 95 | 45 | 0,04 |
| Rührwerkskugelmühle für 11 min. (Vergleich) | | 0,72 |
| Ultraschallaufschluss für 30 s (Vergleich) | | 0,99 |

2.2. Bewahrung der strukturellen Integrität: Abhängigkeit von der Konditioniertemperatur $T_K$

Um die Abhängigkeit der strukturellen Integrität von der Temperatur zu untersuchen, wurden die Folgen von Temperatur-Variationen auf die Entstehung von Ghosts analysiert. Ghosts sind Zellen, deren Metabolite freigesetzt wurden, die ihre strukturelle Integrität jedoch beibehalten haben.

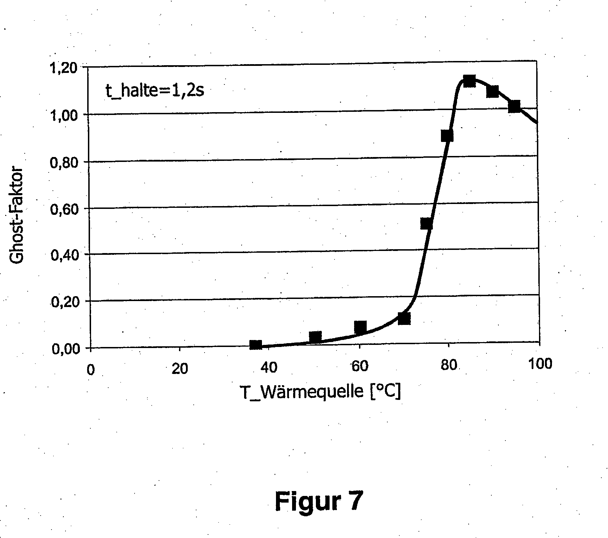

Die Ergebnisse bei einer Konditionierzeit $t_h$ von 1,2 s sind in Figur 7 dargestellt. Während bis zu Konditioniertemperaturen $T_K$ von 75°C nur ein schwacher Anstieg von Ghosts, gemessen am Ghost-Faktor, erfolgt, steigt dieser bei höheren Konditioniertemperaturen sprunghaft an und erreicht bei einer Konditioniertemperatur $T_K$ von etwa 85°C ein Maximum. Bei Konditioniertemperaturen von 90°C und 95°C ist eine geringe Abnahme des Ghost-Faktors zu beobachten, siehe Figur 7. Im Vergleich zu bekannten Metabolit-Freisetzungsverfahren (Kugelmühle, Ultraschall) erzeugt die Freisetzung von Metaboliten mittels des erfindungsgemäßen Verfahrens vorteilhafterweise wesentlich mehr Ghosts als die bekannten Verfahren; dies ist in Tabelle 3 dargestellt.

Tabelle 3 zeigt den Ghost-Faktor bei Probennahme und Metabolit-Freisetzung nach Behandlung der Zellsuspension mittels des integrierten Probennahmesystems, mittels Rührwerkskugelmühle und mittels Ultraschall-Aufschluss. Rührwerkskugelmühle und Ultraschall-Aufschluss zerstören die Zellintegrität und erzeugen eine wesentlich höhere Menge an Zelltrümmern als das integrierte Probennahmesystem. Dagegen erzeugt das erfindungsgemäße Verfahren eine wesentlich größere Menge an Ghost als die Behandlung der Zellsuspension mittels Rührwerkskugelmühle oder Ultraschall.

Tabelle 3:

| Thermische Konditionierung (erfindungsgemäß) | | Ghost-Faktor |
|---|---|---|
| Konditioniertemperatur $T_K$ [°C] | Konditionierzeit $t_h$ [s] | |
| 95 | 1,2 | 1,01 |
| 95 | 20 | 0,57 |
| 95 | 45 | 0,41 |
| Rührwerkskugelmühle für 11 min. (Vergleich) | | -0,05 |
| Ultraschallaufschluss für 30 s (Vergleich) | | -0,12 |

Ein zugrunde liegendes Modell erfordert, dass die Gesamtzellzahl GZZ die Summe aus Trues und Ghosts darstellt, dass sobald keine Trues mehr vorliegen, die Metabolitfreisetzung vollständig sein sollte, dass mit Abnahme der Ghosts die Zahl der Trümmer zunehmen sollte und dass es eine maximale Temperaturgrenze gibt, ab der vermehrt Zelltrümmer und keine Ghosts gebildet werden.

Diese Zusammenhänge wurden experimentell an E. coli für Kultivierungstemperaturen $T_K$ im Bereich von 50°C bis 121°C untersucht. Bei Konditioniertemperaturen über 100°C mussten hierfür andere, das heißt erweiterte, Probennahmevorrichtungen als das erfindungsgemäß bevorzugte eingesetzt werden; um mittels eines angelegten Überdruckes wurde ein Sieden der Probensuspension stets vermieden. Die Konditionierzeit $t_h$ wurde zwischen 1,2 s und 600 s variiert.

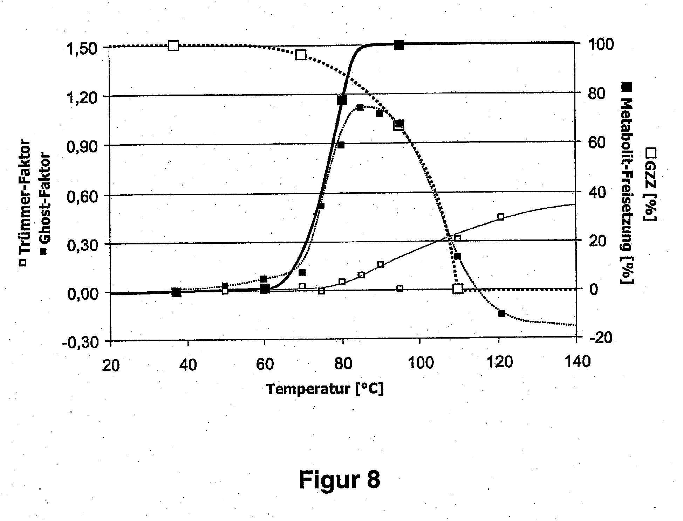

In Figur 8 sind die experimentellen Ergebnisse in der Zusammenschau dargestellt. Eine vollständige Metabolit-Freisetzung erfolgt, wenn alle Trues in Ghosts oder Trümmer umgewandelt wurden. Es konnte gezeigt werden, dass überraschenderweise bei bestimmten Konditioniertemperaturen $T_K$ kritische Schwellenwerte erreicht werden. Der Verlauf des Ghost-Faktors, der ein Maß für die Zahl der gebildeten Ghosts ist, zeigt überraschenderweise ein Optimum bei einem Intervall von $T_K$ von 65°C bis 105°C, insbesondere von 80°C bis 95°C.

Beispiel 3: Kombinatorisches Optimum: Wärmeäquivalente

Wie vorstehend dargestellt, resultiert der Einsatz von Ultraschall oder Rührwerkskugelmühle für die Metabolit-Freisetzung in einem nicht-selektiven Zellaufschluss mit einem hohen Anteil an Trümmern, siehe Tabellen 2 und 3. Durch das erfindungsgemäße Verfahren kann dagegen der Anteil von Trümmern signifikant reduziert, der Anteil von Ghosts bei gleichzeitiger Freisetzung von Metaboliten optimiert werden (Tabelle 3). Aus den vorstehend beschriebenen Untersuchungen ergibt sich, dass die Parameterkombination von Konditionierzeit $t_h$ und Konditioniertemperatur $T_K$ eine große Bedeutung für den Grad der Metabolit-Freisetzung und die Bewahrung der strukturellen Integrität der Zellen hat. Aus den Untersuchungen lässt sich bevorzugt folgendes einfache multifaktorielle Modell ableiten:

(1) Konditioniertemperatur $T_K$ < T_krit,min
Unterschreitet die angelegte Konditioniertemperatur $T_K$ eine kritische Temperatur T_krit,min, so erfolgt aufgrund der Wärmebehandlung nur eine sehr langsame Freisetzung von Metaboliten und nur eine sehr langsame Bildung von Ghosts, wie durch einen tiefen Ghost-Faktor angezeigt.

(2) T_krit,min < Konditioniertemperatur $T_K$ <T_krit,max
und Konditionierzeit $t_h$ < t_krit,min
In diesem Fall erfolgt die Bildung von Ghosts und damit korrelierend die Freisetzung von Metaboliten. Da jedoch die Konditionierzeit die minimale kritische Haltezeit t_krit,min nicht übersteigt, ist die Bildung von Ghosts nicht maximal, es erfolgt keine vollständige Metabolit-Freisetzung.

(3) T_krit,min < Konditioniertemperatur $T_K$ < T_krit,max
und t_krit,min < Konditionierzeit $t_h$ < t_krit,max
Bei diesem Szenario erfolgt eine optimale Bildung von Ghosts bei vollständiger Metabolit-Freisetzung.

(4) T_krit,min < Konditioniertemperatur $T_K$ < T_krit,max
und Konditionierzeit $t_h$ > t_krit,max
Bei diesem Szenario erfolgt zwar eine vollständiger Metabolit-Freisetzung, da jedoch die Konditionierzeit die maximale kritische Haltezeit t_krit,max übersteigt, wird die strukturelle Integrität der Zellen beschädigt und aus den Ghosts entstehen signifikant Trümmer.

(5) Konditioniertemperatur $T_K$ > T_krit,max
Bei diesem Szenario erfolgt ebenfalls eine vollständiger Metabolit-Freisetzung, da jedoch die angelegte Konditioniertemperatur die kritische maximale Temperatur T_krit,max übersteigt, entstehen nur wenige Ghosts. Die Probensuspension ist daher nur sehr schlecht aufzuarbeiten.

Für die kritische minimale und maximale angelegte Temperatur in der durch die Wärmequelle temperierten Kapillare T_krit,min und T_krit,max konnten in Abhängigkeit von den eingesetzten Zellsystemen die in Tabelle 4 dargestellten Werte ermittelt werden.

Tabelle 4 zeigt kritische minimale und maximale angelegte Temperaturen T_krit,min und T_krit,max für verschiedene Zellsysteme: E. coli, B. subtilis und S. cerevisiae.

Tabelle 4:

| Beispielsystem | T_krit,min | T_krit,max |
|---|---|---|
| E. coli | 70°C | Siedepunkt der Probenlösung (105°C) |

(fortgesetzt)

| Beispielsystem | T_krit,min | T_krit,max |
|---|---|---|
| B. subtilis | 75°C | Siedepunkt der Probenlösung (105°C) |
| S. cerevisiae | 70°C | Siedepunkt der Probenlösung (105°C) |

Die kritischen minimalen und maximalen Haltezeiten $t_{krit,min}$ und $t_{krit,max}$ sind keine Konstanten, sondern hängen insbesondere von der angelegten Konditioniertemperatur ab. Um eine optimale Kombination der Parameter Konditionierzeit $t_h$ und Konditioniertemperatur $T_K$ für eine zweckmäßige Dimensionierung der erfindungsgemäßen Vorrichtung zu erreichen, wurden beide Größen über eine Multiplikation kombiniert, das Ergebnis des Produktes als Wärmeäquivalente (WE) mit der Einheit "K·s" bezeichnet. Vor der Multiplikation wurde von der angelegten Konditioniertemperatur $T_K$ [°K] die Kultivierungstemperatur $T_M$ des Organismus [°K] subtrahiert.

Zum Beispiel liegt die Kultivierungstemperatur $T_M$ bei E. coli vorzugsweise bei ca. 310°K; dies entspricht ca. 37°C. Das Optimum an WE konnte so für E. coli bei $110 \pm 20$ K·s ermittelt werden (Tabelle 5).

Für weitere biologische Systeme sind repräsentativ in Tabelle 5 weitere gefundene Optima (WE_opt) angegeben.

Tabelle 5:

| Beispielsystem für | | WE_min | WE_opt | WE_max |
|---|---|---|---|---|
| E. coli | gram-negative Prokaryonten | 70 | 110 | 300 |
| B. subtilis | gram-positive Prokaryonten | 90 | 130 | 300 |
| S. cerevisiae | Eukaryonten | 70 | 110 | 260 |

Beispiel 4: Geometrie des integrierten Probennahmesystems

Aus dem Wissen um die Abhängigkeit von der Größe "Wärmeäquivalent" als eine Funktion der Konditionierzeit $t_h$, Konditioniertemperatur $T_K$ und Kultivierungstemperatur $T_M$ ergeben sich für die geometrische Auslegung zwingende geometrische Randbedingungen und Kriterien, deren Einhaltung für die (i.) ausreichend schnelle quantitative Metabolitfreisetzung (Schritte "Transfer" durch das Totvolumen sowie "Quenching"), wobei die Zeitkonstanten der biochemischen Reaktionen (turnover) zu berücksichtigen sind, und (ii.) der Erzielung von Zellen der gewünschten Struktur, das heißt insbesondere "Ghost-Zellen", durch die Wahl charakteristischer Konditionierzeiten unbedingt erforderlich ist. Eine weitere Randbedingung ergibt sich (iii.) aus den Anforderungen der subsequenten Analyse der Proben bezüglich des minimalen Probennahmevolumens (Volumenstrom).

Die geometrische Auslegung muss gewährleisten, dass die ermittelte kritische Temperatur T_krit,min<T_krit<T_krit,max (Schritt "Quenching") innerhalb der Heizzeit t_heiz auf dem Streckenabschnitt s_heiz erreicht wird. Hierbei ist die für das Quenching des Metabolismus erforderliche Zeitkonstanten t_heiz maßgebend.

Ausgehend von einer Bilanz der Wärmeströme lassen sich geometrische Beziehungen ableiten:

$$\rho \cdot \dot{V} \cdot c_p \cdot (T_{Suspension} - T_{WT}) = -Nu \cdot \lambda \cdot \pi \cdot s_{heiz} \cdot \frac{(T_{WT} - T_{Suspension}) - (T_{WT} - (T_{WT} - 5K))}{\ln \frac{(T_{WT} - T_{Suspension})}{(T_{WT} - (T_{WT} - 5K))}} \quad \text{(Gl.1)}$$

T_WT ist in unserem System gleichzusetzen mit T_Wärmequelle. Unter Verwendung einer geeigneten Kriteriengleichung der Form $Nu = c \times Re^m \times Pr^n$ für die Nusselt-Zahl (VDI-Wärmeatlas) folgt (Gl.2):

$$\rho \cdot \dot{V} \cdot c_p \cdot (T_{Suspension} - T_{W\ddot{a}rmequell}) = -(3.953 + 0.0864 [1 + 0.8 \cdot (\frac{d}{D})^{0.9}] \cdot (\frac{4 \cdot \dot{V}}{\pi \cdot d \cdot v})^{0.652} \cdot 3.4^{\frac{1}{3}}) \cdot \lambda \cdot \pi \cdot s_{heiz} \cdot 58K$$

Im Folgenden sind Stoffdaten (auf der Basis von Wasser, temperaturgemittelt), dimensionslose Kenngrößen Re und Pr zur Berechnung von Fluiddynamik und Wärmeübergang sowie geometrische Größen (d, D) des verwendeten Probennahmesystems aufgelistet:

| | |
|---|---|
| Re | 4900 |
| Re=0,5*[Re(35°C)+Re(90°C)] Pr | 3,4 |
| Pr=0,5*[Pr(35°C)+Pr(90°C)] c/m/n, mit m = m(d, D) | 0,083/0,652/0,333 |
| d | 2,03 mm |
| D | 57 mm |
| w | 1,08 m/s |
| ΔTm | 58 K |
| Q̇ | 761 W |

Mit den auf der Basis von Wasser temperaturgemittelten Stoffwerten (p = 0.979 mg/l, cp = 4,192 kJ/(kg K), $\nu$ = 0,525 x 10$^{-6}$ m$^2$/s, $\lambda$= 0,652 W/(m K) sowie als technischen Randbedingungen eine mindestens erforderliche Probennahmefrequenz von f = 5 s$^{-1}$ (entsprechender Volumenstrom von mindestens 2,5 ml/s) und ein Windungsdurchmesser der Rohrwendel von 0,057m (siehe Tabelle 6 für Stoffdaten, dimensionslose Kennzahlen zur Berechnung von Fluiddynamik und Wärmeübergang sowie den geometrischen Größen d, D) folgt für die geometrische Auslegung zunächst folgende Gleichung (Gl.3):

$$s_{heiz} = \frac{1}{0.618 + 0.02 \cdot [1 + 0.8 \cdot (\frac{d}{0.057})^{0.9}] \cdot (\frac{6.06}{d})^{0.5 + 0.2903 \cdot (d/0.057)^{0.194}}}$$

Die Anzahl der zu übertragenden Wärmeäquivalente durch das integrierte Probennahmesystem ist eine Funktion der verwendeten Organismen, d.h. der spezifischen intrazellulären Metabolit-Poolkonzentrationen, und hat durch eine geeignete Dimensionierung derart zu erfolgen, dass in der für den Vorgang Transport durch das Totvolumen und den Vorgang Quenching benötigten Zeit maximal 10% dieser Poolkonzentration auf der Probennahmestrecke umgesetzt werden kann. Ein Verlust von 10% der Poolkonzentrationen kann im Hinblick auf quantitative Analysen gerade noch akzeptiert werden. Wegen des hohen turnover wurde für die Berechnungen der ATP-turnover herangezogen. Intrazelluläre ATP-Konzentrationen, spezifischen ATP-Bildungsraten und turnover $\tau$ für die betrachteten Organismen sind in Tabelle 6 zusammengefasst.

Die Tabelle 6 zeigt Intrazelluläre ATP-Konzentrationen, spezifischen ATP-Bildungsraten und turnover $\tau$ für verschiedene Zellsysteme. Das Zellsystem E. coli repräsentiert gram-negative Prokaryonten, B. subtilis gram-positive Prokaryonten, S. cerevisiae einzellige eukaryotische Systeme. Extremwerte sind $\tau$ATP = 0,1 s und $\tau$ATP = 3 s.

Tabelle 6:

| Verdünnungsrate D = 0,1 h⁻¹ | | | |
|---|---|---|---|
| Zellsystem | cATP [μmol/gTS] | qATP [mmol/(gTS h)] | τATP [s] |
| E. coli | 0,7-7,6 | 13-25 | 0,1-2,1 |
| B. subtilis | 1,8 | 15,5 | 0,4 |
| S. cerevisiae | 0,4-8,0 | 10,8 | 0,1-2,7 |
| | | | |
| Verdünnungsrate D = 0,4 h⁻¹ | | | |
| Zellsystem | cATP [μmol/gTS] | qATP [mmol/(gTS h)] | τATP [s] |
| E. coli | 8,7 | 60,5 | 0,5 |
| B. subtilis | 1,8 | 55,2 | 0,1 |
| S. cerevisiae | 0,4-8,0 | 69,8 | ca. 0,4 |

Dann gilt (Gl.4):

$$d \approx \left( \frac{4 \cdot \dot{V} \cdot \dfrac{0.1 \cdot c_{ATP}}{q_{turnover(ATP)}}}{\pi \cdot s_{heiz}} \right)^{0.5}$$

Die Lösung der Wärmebilanz gemäß Gl.1 lieferte die für die Übertragung der Wärmeäquivalente erforderliche Heizstrecke s_heiz des Vorgangs Quenching. Diese ist eine Funktion des Rohrwendel-Innendurchmessers nach Gl.3 und Gl.4.

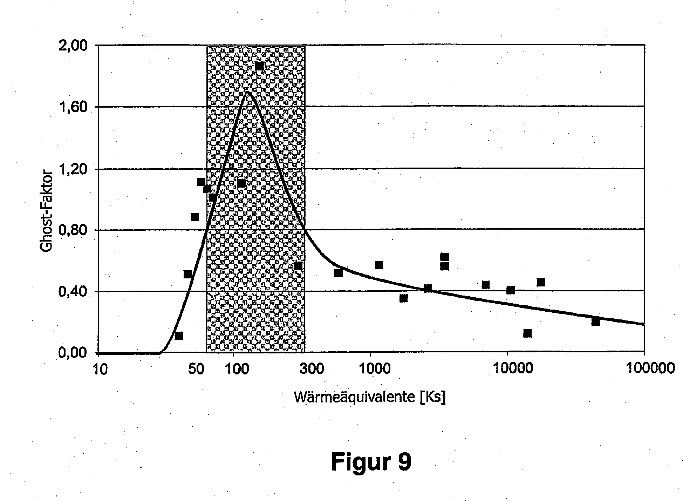

Ist die kritische Temperatur T_krit,min<T_krit<T_krit,max erreicht, müssen auf der Probennahmestrecke s_halte die Wärmeäquivalente übertragen werden (Figur 9). Die von der Geometrie abhängige Zeit t_halte ergibt sich zu (Gl.5):

$$t_{halte} = \frac{WE_{opt}}{T_{W\ddot{a}rmequelle} - T_{Suspension}} - \frac{\pi \cdot d^2}{\dot{V}\left( 2.472 + 0.08 \cdot [1 + 0.8 \cdot (\frac{d}{0.057})^{0.9}] \cdot \left(\frac{6.06}{d}\right)^{0.5 + 0.2903 \cdot (d/0.057)^{0.194}} \right)}$$

Für die Haltestrecke s_halte gilt (Gl. 6):

s_halte = t_halte · w

und damit für die gesamte temperierte Strecke s_Wärmequelle (Gl. 7):

s_Wärmequelle = s_halte+s_heiz

Eine bevorzugte apparative Umsetzung ist insbesondere in Form einer Rohrwendel mit den charakteristischen geometrischen Abmessungen wie nachstehend dargestellt, erfüllbar:

Rohrwendel- Innendurchmesser:  $d\_i = 2.8$ mm - 2.2/ +1.7 mm

Heizstrecke:  $s\_heiz = 16$ cm $\pm$ 6 cm

Geometriekennzahl:  $d\_i/s\_heiz = 0.0175 \pm 0.0135$

temperierte Kapillarstrecke:  $s\_Wärmequelle = 240$ cm -160/ +1271 cm

Die Abweichungen der einzelnen geometrischen Größen vom angegebenen optimalen Wert können sich je nach Zellsystem und Kultivierungsbedingungen, spezifischen Turnoverzeiten (vgl. Tabelle 6) sowie je nach Volumenstrom, der bevorzugt zwischen 2,5 und 8 ml/s eingestellt wird, ergeben. Bei kleinen Innendurchmessern von $d\_i < 1{,}2$ mm ist bei Verwendung von GI. 3 der Windungsdurchmesser der Rohrwendel vorzugsweise zu verkleinern (lt. VDI-Wärmeatlas).

Symbolverzeichnis:

| Symbol | Bezeichnung | Einheit |
|---|---|---|
| $\dot{V}$ | Volumenstrom durch integrierte Probennahme | $m^3/s$ |
| f | Probennahmefrequenz | $s^{-1}$ |
| $\tau$ | turnover | s |
| $\rho$ | Dichte | $kg/m^3$ |
| w | mittlere Strömungsgeschwindigkeit | |
| $d\_i$ | Rohrwendel-Innendurchmesser | m |
| D | Windungsdurchmesser | m |
| $\Delta Tm$ | mittlere logarithmische Temperaturdifferenz | K |
| Re | Reynolds-Zahl | |
| Pr | Prandtl-Zahl | |
| Nu | Nusselt-Zahl | |
| c,m,n | Koeffizienten bzw. Exponenten | |
| cp | spezifische Wärmekapazität | $kJ/(kg\ K)$ |
| $\nu$ | kinematische Zähigkeit | $m^2/s$ |
| $\eta$ | dynamischen Zähigkeit | $kg/(m\ s)$ |
| $\lambda$ | Wärmeleitzahl | $W/(m\ K)$ |

Beispiel 5: Gewinnung und Analyse intrazellulärer Metabolite

Der E.coli-Stamm K-12 wurde steril in einem Bioreaktor (KLF 2000, Bioengineering, Wald, Schweiz) bei einer Kultivierungstemperatur $T_M$ (entspricht T_suspension) von 37°C, entsprechend 310°K, kultiviert. Unter Verwendung der erfindungsgemäßen Vorrichtung einer integrierten Probennahme, die mit folgenden Parametern betrieben wurde, wurden Proben konditioniert:

| | |
|---|---|
| Volumenstrom | 3,5 ml/s |
| Kapillarinnendurchmesser, $d\_i$ | 2,03 mm |
| Länge der temperierten Kapillarstrecke, $s\_Wärmequelle$ | 205 cm |
| Konditioniertemperatur $T_K$, T_Wärmequelle | 95°C |

Bei einer Konditioniertemperatur $T_K$ von 95°C und einer Konditionierzeit $t_h$ von 1,9 s wurde erfindungsgemäß ein Wärmeäquivalent WE von 110 Ks übertragen.

Ergebnis I:

Die Zellsuspension konnte während der Probennahme simultan gequencht und selektiv aufgeschlossen werden.
Die Analyse der aus den konditionierten Zellen freigesetzten Metabolite erfolgte mittels Ionenchromatographie (Dionex DX500) und LC-MS (Thermo Quest Finnigan AQA) nach einem einfachen Filtrationsschritt mit einem Poren-durchmesser von 0,2 μm), worin die Zellhüllen (Ghosts) und eventuell noch vorhandene intakte Zellen (Reals), zurückgehalten wurden.

Durch wiederholte erfindungsgemäße Probennahme wurde der zeitliche Verlauf ausgewählter intrazellulärer Metabolitkonzentrationen einer steady-state E. coli-Kultur bei $D=0,1$ $h^{-1}$ mit der Biomassekonzentration im Bioreaktor von $c=2,30$ g/l (in g Trockengewicht) beobachtet, nachdem zum Zeitpunkt $t=0$ s ein extrazelluläre Puls von 0,3 g/l Glucose in den Bioreaktor gegeben wurde. Gemessen wurden beispielhaft PEP = Phosphoenolpyruvat, DHAP/GAP = Summe von Dihydroxyacetonphosphat und Glycerinaldehyd-3-phosphat, ADP = Adenosindiphosphat, 2PG/3PG = Summe aus 2-Phosphoglycerat und 3-Phosphoglycerat sowie FBP = Fructosebisphosphat.

Ergebnis II:

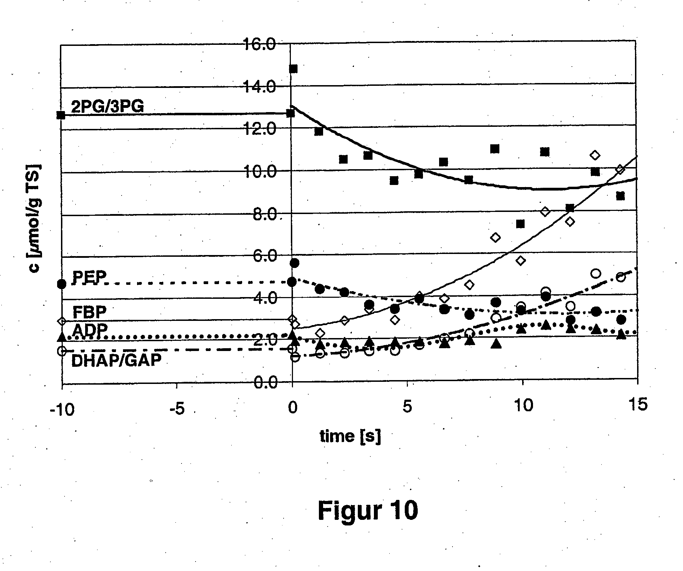

Die Figur 10 zeigt das Ergebnis der Analyse der erfindungsgemäß bei der Probennahme und -überführung freigesetzten Metabolite ausgewählter Stoffklässen. Legende: PEP = Phosphoenolpyruvat, DHAP/GAP = Summe von Dihydroxyacetonphosphat und Glycerinaldehyd-3-phosphat, ADP = Adenosindiphosphat, 2PG/3PG = Summe aus 2-Phosphoglycerat und 3-Phosphoglycerat, FBP = Fructosebisphosphat.

**Patentansprüche**

1. Verfahren zum thermischen Konditionieren einer biologischen Zelle, wobei die Zelle bei einer Kultivierungstemperatur $T_M$ in einem Kulturmedium kultiviert wird und anschließend für eine Konditionierzeit $t_h$ bei einer Konditionier-temperatur $T_K$ konditioniert wird, **dadurch gekennzeichnet, dass** das Wärmeäquivalent **WE,** gemäß der Formel

$$WE = t_h \cdot (T_K - T_M)$$

von 90 K·s bis 150 K·s und die Konditioniertemperatur $T_K$ von 80°C bis 95°C beträgt, wobei die Zellstruktur der thermisch konditionierten biologischen Zelle erhalten bleibt.

2. Verfahren nach Anspruch 1, wobei die Konditionierzeit $t_h$ von 1 s bis 180 s, beträgt.

3. Verfahren nach einem der vorstehenden Ansprüche, wobei die Kultivierungstemperatur $T_M$ von 26°C bis 42°C beträgt.

4. Verfahren nach einem der vorstehenden Ansprüche, wobei die biologische Zelle ein gram-negativer Prokaryont wie E. coli oder ein Eukaryont wie S. cerevisiae ist und das Wärmeäquivalent **WE** $110 \pm 20$ K·s beträgt.

5. Verfahren nach einem der vorstehenden Ansprüche, wobei die Zelle ein gram-positiver Prokaryont wie B. subtilis ist und das Wärmeäquivalent **WE** $130 \pm 20$ K·s beträgt.

6. Verfahren nach einem der vorstehenden Ansprüche, wobei das Kulturmedium ein flüssiges Medium ist und das thermische Konditionieren dadurch erfolgt, dass das die biologische Zelle enthaltende flüssige Medium in einer Kapillare fließt, wobei sich die Zelle für eine Konditionierzeit $t_h$ innerhalb einer auf die Konditioniertemperatur $T_K$ temperierten Kapillarstrecke der Kapillare befindet.

7. Verfahren nach Anspruch 6, wobei der Volumenstrom in der temperierten Kapillarstrecke von 0,5 ml/s bis 12 ml/s beträgt.

8. Verfahren nach einem der vorstehenden Ansprüche, wobei der Schritt der thermischen Konditionierung mit der Überführung des die biologischen Zellen enthaltenden Kulturmediums aus einem Kultivierungsgefäß, insbesondere einem Bioreaktor, in ein Auffanggefäß, insbesondere ein Probensammelgefäß, durchgeführt wird.

9. Verfahren zur Gewinnung eines Inhaltsstoffs aus einer biologischen Zelle, enthaltend die Schritte:

   a) Kultivieren der Zelle in einem Kulturmedium,
   b) thermisches Konditionieren der Zelle gemäß dem Verfahren nach einem der Ansprüche 1 bis 8, wobei der Inhaltsstoff aus der thermisch konditionierten Zelle freigesetzt wird, und
c) Isolieren des freigesetzten Inhaltsstoffs aus dem Kulturmedium.

10. Verfahren zum quantitativen Nachweis eines Inhaltsstoffs in einer biologischen Zelle, enthaltend die Schritte:

a) Kultivieren der Zelle in einem Kulturmedium,
b) thermisches Konditionieren der Zelle gemäß dem Verfahren nach einem der Ansprüche 1 bis 8, wobei der Inhaltsstoff aus der thermisch konditionierten Zelle freigesetzt wird, und
c) quantitativer Nachweis des freigesetzten Inhaltsstoffs im Kulturmedium.

11. Verfahren zum qualitativen Nachweis eines Inhaltsstoffs in einer biologischen Zelle, enthaltend die Schritte:

a) Kultivieren der Zelle in einem Kulturmedium,
b) thermisches Konditionieren der Zelle gemäß dem Verfahren nach einem der Ansprüche 1 bis 8, wobei der Inhaltsstoff aus der thermisch konditionierten Zelle freigesetzt wird, und
c) qualitativer Nachweis des freigesetzten Inhaltsstoffs im Kulturmedium.

12. Verfahren nach einem der vorstehenden Ansprüche, wobei der Inhaltsstoff ein intrazellulärer Metabolit, ausgewählt aus der Gruppe der Aminosäuren, Aminen und deren Derivate, der Carboxylsäuren, der Alkohole, der Aldehyde, der Ketone, der Phosphatester (ohne die Nukleinsäuren), der Nukleinsäuren und verwandter Verbindungen, der Zucker und verwandter Verbindungen, der Lipide, der Steroide, der Fettsäuren, der Vitamine, der Coenzyme und der anorganischen Ionen ist.

13. Zellkonditioniervorrichtung mit Probenüberführungsstrecke (5), Probensammelvorrichtung (6) und mindestens einer an einer Wärmequelle (4) angeordneten und vom flüssigen Kulturmedium durchflossenen Kapillare (3) mit einem Innendurchmesser von 0,5 mm bis 4,5 mm wobei die Kapillare entlang einer temperierten Kapillarstrecke von 80 cm bis 1510 cm mit der Wärmequelle in Kontakt steht und speziell ausgebildet ist für ein zu übertragendes Wärmeäquivalent von 90 Ks bis 150 Ks bei einer Konditioniertemperatur $T_K$ von 80°C bis 95°C, wobei die Zellstruktur einer thermisch konditionierten Zelle erhalten bleibt, indem

a) für einen kleinen Volumenstrom von etwa 2,5 ml/s

i. bei einer unteren Umsatzrate des Metaboliten von etwa 0,1 s der Innendurchmesser der Kapillare etwa 0,5 mm,
ii. bei einer oberen Umsatzrate des Metaboliten von etwa 2,7 s der Innendurchmesser der Kapillare etwa 2 mm und

b) für einen großen Volumenstrom von etwa 8 ml/s

i. bei einer unteren Umsatzrate des Metaboliten von etwa 0,1 s der Innendurchmesser der Kapillare etwa 1 mm,
ii. bei einer oberen Umsatzrate des Metaboliten von etwa 2,7 s der Innendurchmesser der Kapillare etwa 4 mm
beträgt.

14. Zellkonditioniervorrichtung nach Anspruch 13, wobei die Zellkonditioniervorrichtung als Probennahmevorrichtung zur Entnahme einer Probe aus einer Quelle (1) eines die Zelle enthaltenden flüssigen Kulturmediums (2) ausgebildet ist.

15. Verwendung der Zellkonditioniervorrichtung gemäß Anspruch 13 oder 14 zur Gewinnung eines Inhaltsstoffs aus einer biologischen Zelle.

16. Verwendung der Zellkonditioniervorrichtung gemäß Anspruch 13 oder 14 zum quantitativen Nachweis eines Inhaltsstoffs in einer biologischen Zelle.

17. Verwendung der Zellkonditioniervorrichtung gemäß Anspruch 13 oder 14 zum qualitativen Nachweis eines Inhaltsstoffs in einer biologischen Zelle.

**Claims**

1. A method of thermal conditioning of a biological cell, wherein the cell is cultured in a culture medium at a culturing temperature $T_M$ and subsequently conditioned at a conditioning temperature $T_K$ for a conditioning time $t_h$, **characterized in that** the thermal equivalent WE, expressed by the formula

$$WE = t_h \cdot (T_K - T_M),$$

is in the range of 90 K· sec to 150 K· sec and the conditioning temperature $T_K$ is 80 °C to 95 °C, wherein the cell structure of the thermally conditioned biological cell is maintained.

2. Method according to claim 1, wherein the conditioning time $t_h$ is 1 sec to 180 sec.

3. Method according to one of the preceding claims, wherein the culturing temperature $T_M$ is 26 °C to 42 °C.

4. Method according to one of the preceding claims, wherein the biological cell is a gram-negative prokaryote such as E. coli, or a eukaryote such as S. cerevisiae, and the thermal equivalent WE is 110 $\pm$ 20 K· sec.

5. Method according to one of the preceding claims, wherein the cell is a gram-positive prokaryote such as B. subtilis, and the thermal equivalent WE is 130 $\pm$ 20 K· sec.

6. Method according to one of the preceding claims, wherein the culture medium is a liquid medium, and wherein thermal conditioning occurs by the liquid medium containing the biological cell flowing through a capillary, wherein the cell is disposed in a temperature-controlled segment of the capillary at the conditioning temperature $T_K$, for a conditioning time $t_h$.

7. Method according to claim 6, wherein the volumetric flow in the temperature-controlled segment of the capillary is 0.5 mL/sec to 12 mL/sec.

8. Method according to one of the preceding claims, wherein thermal conditioning is carried out with the transfer of the culture medium containing the biological cells from a culturing vessel, in particular a bioreactor, into a receiving vessel, in particular a sample collection vessel.

9. A method of recovering a component material from a biological cell, comprising the following steps:

   (a) culturing the cell in a culture medium;
   (b) thermally conditioning the cell according to the method of one of claims 1 to 8, wherein the component material is liberated from the thermally conditioned cell; and
   (c) isolating the liberated component material from the culture medium.

10. A method of quantitative determination of a component material in a biological cell, comprising the following steps:

   (a) culturing the cell in a culture medium;
   (b) thermally conditioning the cell according to the method of one of claims 1 to 8, wherein the component material is liberated from the thermally conditioned cell; and
   (c) quantitatively determining the liberated component material in the culture medium.

11. A method of qualitative determination of a component material in a biological cell, comprising the following steps:

   (a) culturing the cell in a culture medium;
   (b) thermally conditioning the cell according to the method of one of claims 1 to 8, wherein the component material is liberated from the thermally conditioned cell;
   (c) qualitatively determining the liberated component material in the culture medium.

12. Method according to one of the preceding claims, wherein the component material is an intracellular metabolite, selected from the group consisting of amino acids, amines and their derivatives, carboxylic acids, alcohols, aldehydes, ketones, phosphate esters (other than nucleic acids), nucleic acids and congeners, sugars and congeners, lipids, steroids, fatty acids, vitamins, coenzymes, and inorganic ions.

13. An apparatus for conditioning of cells, having a sample-transferring segment (5), a sample-collecting device (6), and at least one capillary (3) which is disposed adjacent to a heat source (4), through which capillary a liquid culture medium is flowed, the capillary having an interior diameter of 0.5 mm to 4.5 mm, wherein the capillary remains in contact with the heat source along a temperature-controlled capillary segment having a length of 80 cm to 1510 cm, and is especially configured for a thermal equivalent of 90 K· sec to 150 K· sec which is to be transferred at a conditioning temperature $T_K$ of 80°C to 95°C, wherein the cell structure of a thermally conditioned cell is maintained, by

a) for a low volumetric flow rate of about 2.5 mL/sec

i. at a lower conversion rate of the metabolite of about 0.1 sec, the interior diameter of the capillary is about 0.5 mm;
ii. at an upper conversion rate of the metabolite of about 2.7 sec, the interior diameter of the capillary is about 2 mm; and

b) for a high volumetric flow rate of about 8 mL/sec

i. at a lower conversion rate of the metabolite of about 0.1 sec, the interior diameter of the capillary is about 1 mm;
ii. at an upper conversion rate of the metabolite of about 2.7 sec, the interior diameter of the capillary is about 4 mm.

14. Apparatus for conditioning cells according to claim 13, wherein the cell conditioning apparatus is in the form of a sample-taking device for sampling from a source (1) of a liquid culture medium (2) containing the cell.

15. Use of the cell conditioning apparatus according to claim 13 or 14 for recovering a component material from a biological cell.

16. Use of the cell conditioning apparatus according to claim 13 or 14 for quantitative determination of a component material in a biological cell.

17. Use of the cell conditioning apparatus according to claim 13 or 14 for qualitative determination of a component material in a biological cell.

## Revendications

1. Procédé de conditionnement thermique d'une cellule biologique, dans lequel la cellule est cultivée à une température de culture $T_M$ dans un milieu de culture, et est ensuite conditionnée pendant une durée de conditionnement $t_h$ à une température de conditionnement $T_K$, **caractérisé en ce que** l'équivalent thermique WE selon la formule

$$WE = t_h \cdot (T_K - T_M)$$

va de 90 K· s à 150 K· s et la température de conditionnement $T_K$ va de 80°C à 95°C, dans lequel la structure cellulaire de la cellule biologique conditionnée de manière thermique est conservée.

2. Procédé selon la revendication 1, dans lequel la durée de conditionnement $t_h$ va de 1 s à 180 s.

3. Procédé selon l'une quelconque des revendications précédentes, dans lequel la température de culture $T_M$ va de 26°C à 42°C.

4. Procédé selon l'une quelconque des revendications précédentes, dans lequel la cellule biologique est un procaryote Gram négatif comme E. coli ou un eucaryote comme S. cerevisiae et l'équivalent thermique WE se monte à 110+/- 20 K·s.

**EP 1 720 976 B1**

5. Procédé selon l'une quelconque des revendications précédentes, dans lequel la cellule est un procaryote Gram positif comme B. subtilis et l'équivalent thermique WE se monte à 130 +/- 20 K · s.

6. Procédé selon l'une quelconque des revendications précédentes, dans lequel le milieu de culture est un milieu liquide et le conditionnement thermique s'effectue en ce que le milieu liquide contenant la cellule biologique s'écoule dans un tube capillaire, dans lequel la cellule se trouve pendant une durée de conditionnement $t_h$ au sein d'un trajet capillaire du tube capillaire tempéré à la température de conditionnement $T_K$.

7. Procédé selon la revendication 6, dans lequel le courant volumique dans le trajet capillaire tempéré va de 0,5 ml/s à 12 ml/s.

8. Procédé selon l'une quelconque des revendications précédentes, dans lequel l'étape du conditionnement thermique est effectuée avec le transfert du milieu de culture contenant les cellules biologiques d'un récipient de culture, notamment d'un bioréacteur, vers un récipient collecteur, notamment un récipient de stockage d'échantillon.

9. Procédé d'obtention d'une substance à partir d'une cellule biologique, contenant les étapes :

   a) culture de la cellule dans un milieu de culture,
   b) conditionnement thermique de la cellule conformément au procédé selon l'une quelconque des revendications 1 à 8, dans lequel la substance est libérée de la cellule conditionnée de manière thermique, et
   c) isolation de la substance libérée du milieu de culture.

10. Procédé de détermination quantitative d'une substance dans une cellule biologique, contenant les étapes :

   a) culture de la cellule dans un milieu de culture,
   b) conditionnement thermique de la cellule conformément au procédé selon l'une quelconque des revendications 1 à 8, dans lequel la substance est libérée de la cellule conditionnée de manière thermique, et
   c) détermination quantitative de la substance libérée dans le milieu de culture.

11. Procédé de détermination qualitative d'une substance dans une cellule biologique, contenant les étapes :

   a) culture de la cellule dans un milieu de culture,
   b) conditionnement thermique de la cellule conformément au procédé selon l'une quelconque des revendications 1 à 8, dans lequel la substance est libérée de la cellule conditionnée de manière thermique, et
   c) détermination qualitative de la substance libérée dans le milieu de culture.

12. Procédé selon l'une quelconque des revendications précédentes, dans lequel la substance est un métabolite intracellulaire sélectionné parmi le groupe des acides aminés, amines et leurs dérivés, des acides carboxyliques, des alcools, des aldéhydes, des cétones, des esters de phosphate (sans les acides nucléiques), des acides nucléiques et composés apparentés, des sucres et composés apparentés, des lipides, des stéroïdes, des acides gras, des vitamines, des co-enzymes et des ions inorganiques.

13. Dispositif de conditionnement cellulaire avec trajet de transfert d'échantillon (5), dispositif de stockage d'échantillon (6) et au moins un tube capillaire (3) disposé sur une source de chaleur (4) et parcouru par le milieu de culture liquide avec un diamètre interne de 0,5 mm à 4,5 mm, dans lequel le tube capillaire est en contact avec la source de chaleur le long d'un trajet capillaire tempéré de 80 cm à 1510 cm et est spécialement réalisé pour un équivalent thermique à transmettre de 90 Ks à 150 Ks à une température de conditionnement $T_K$ de 80°C à 95°C, dans lequel la structure cellulaire d'une cellule conditionnée de manière thermique est conservée en ce que

   a) pour un petit courant volumique d'environ 2,5 ml/s

      i. à une vitesse de conversion inférieure du métabolite d'environ 0,1 s, le diamètre interne du tube capillaire se monte à environ 0,5 mm,
      ii. à une vitesse de conversion supérieure du métabolite d'environ 2,7 s, le diamètre interne du tube capillaire se monte à environ 2 mm, et

   b) pour un grand courant volumique d'environ 8 ml/s

24

i. à une vitesse de conversion inférieure du métabolite d'environ 0,1 s, le diamètre interne du tube capillaire se monte à environ 1 mm,

ii. à une vitesse de conversion supérieure du métabolite d'environ 2,7 s, le diamètre interne du tube capillaire se monte à environ 4 mm.

14. Dispositif de conditionnement cellulaire selon la revendication 13, dans lequel le dispositif de conditionnement cellulaire est réalisé en tant que dispositif de prélèvement d'échantillon pour le prélèvement d'un échantillon à partir d'une source (1) d'un milieu de culture liquide (2) contenant la cellule.

15. Utilisation du dispositif de conditionnement cellulaire selon la revendication 13 ou 14 pour l'obtention d'une substance à partir d'une cellule biologique.

16. Utilisation du dispositif de conditionnement cellulaire selon la revendication 13 ou 14 pour la détermination quantitative d'une substance dans une cellule biologique.

17. Utilisation du dispositif de conditionnement cellulaire selon la revendication 13 ou 14 pour la détermination qualitative d'une substance dans une cellule biologique.

**Figur 1**

**Figur 2**

**Figur 3**

**Figur 4**

**Figur 5**

**Figur 6**

**Figur 7**

**Figur 8**

**Figur 9**

**Figur 10**

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente

- DE 4407439 A1
- DE 19705289 A1
- EP 0722075 A1
- DE 2345243 A1
- WO 9216807 A
- EP 0217662 A1
- US 6197553 B1

### In der Beschreibung aufgeführte Nicht-Patentliteratur

- **GERHARDT, P. ; R. G. E. MURRAC ; W. A. WOOD ; N. R. KRIEG.** Methods for general and molecular bacteriology. American Society for Microbiology, 1994
- **SAMBROOK, J. ; E. F. FRITSCH ; T. MANIATIS.** Molecular cloning: A laboratory manual. Cold Spring Harbor, 1989
- **VON THEOBALD ; MITARBEITERN.** In vivo analysis of glucose-induced fast changes in yeast adenine nucleotide pool applying a rapid sampling technique. *Anal Biochem.,* 1993, vol. 214, 31-7
- **VON LANGE ; MITARBEITERN.** Improved rapid sampling for in vivo kinetics of intracellular metabolites in Saccharomyces cerevisiae. *Biotechnol. Bioeng.,* 2001, vol. 75, 406-15
- **BHATTACHARYA ; FUHRMAN ; INGRAM ; NICKERSON ; CONWAY.** Single-run separation and detection of multiple metabolic intermediates by anion-exchange high-performance liquid chromatography and application to cell pool extracts prepared from Escherichia coli. *Anal. Biochem.,* 1995, vol. 232, 98-106
- Tran & Unden Changes in the proton potential and the cellular energetics of Escherichia coli during growth by aerobic and anaerobic respiration or by fermentation. *Eur. J. Biochem.,* 1998, vol. 251, 538-43